# EUROPÄISCHE PATENTSCHRIFT

(11) **EP 0 912 164 B1**
(45) Veröffentlichungstag und Bekanntmachung des Hinweises auf die Patenterteilung: **06.02.2002**
(21) Anmeldenummer: 97931792.2
(22) Anmeldetag: 07.07.1997
(51) Int. Cl.: A61K 7/48, A61K 7/06, A61K 7/16, C11D 3/22

(54) **STÄRKEHALTIGE REINIGUNGS- UND PFLEGEMITTEL**
STARCHY CLEANING AND COSMETIC CARE PREPARATIONS
PRODUITS DE NETTOYAGE ET DE SOINS DE BEAUTE CONTENANT DE L'AMIDON

(30) Priorität: 08.07.1996 DE 19627498
(43) Veröffentlichungstag der Anmeldung: 06.05.1999
(73) Patentinhaber: NATIONAL STARCH AND CHEMICAL INVESTMENT HOLDING CORPORATION, Wilmington, Delaware 19850 (US)
(72) Erfinder: MÜLLER, Wilfried, D-52531 Übach-Palenberg (DE); VATHJE, Rainer, D-52223 Stolberg (DE); CARDINALI, Martin, Scott, Martinsville, NJ 08836 (US)
(74) Vertreter: Hagemann, Heinrich, Dr.rer.nat., Dipl.-Chem.
(86) Internationale Anmeldenummer: EP9703581
(87) Internationale Veröffentlichungsnummer: WO9801109

(56) Entgegenhaltungen:
- EP-A- 0 487 000
- EP-A- 0 489 424
- WO-A-89/04842
- US-A- 4 508 705

## Beschreibung

Die vorliegende Erfindung betrifft Mittel zur Reinigung oder Pflege von Haut, Zähnen oder Haar oder zur Reinigung von glatten Oberflächen. Die Erfindung betrifft demzufolge Mittel, die bei bestimmungsgemäßer Anwendung mit der menschlichen Haut in Berührung kommen oder kommen können.

In Kosmetika sind feuchtigkeitsspendende Eigenschaften, taktile Eigenschaften, die ein weiches Gefühl auf Haut und Lippen vermitteln, und eine glatte, leicht verstreichbare Textur erstrebenswert. Die taktilen Eigenschaften werden in herkömmlichen kosmetischen Zusammensetzungen durch hydrophobe Bestandteile wie Wachse, Öle und Fette, verliehen.

Verbesserte feuchtigkeitsspendende Eigenschaften werden erhalten, indem man der Zusammensetzung hydrophile Bestandteile zufügt und eine stabile Wasser-in-Öl-Emulsion bildet. Die im Stand der Technik bekannten emulsionsförmigen Zusammensetzungen weisen verschiedene Nachteile, z.B. eine mangelnde Emulsionsstabilität und einen hohen Preis der Bestandteile (z.B. Hyaluronsäure), insbesondere bei Creme-, Lotions- und Gesichtsseifenzusammensetzungen, auf.

Es sind im Stand der Technik Kosmetika bekannt, die zur Überwindung dieser Nachteile bestimmte Stärken enthalten.

Die US 5 279 313 beschreibt eine Haarbleichmittelzusammensetzung, die ein Persulfat einer bestimmten Teilchengröße und alkalisch reagierende Salze enthält. Die Zusammensetzung kann als wasserlösliches Verdickungsmittel Stärken enthalten. Als Beispiele werden Maisstärke, Stärkeether, wie Carboxymethylstärke, Hydroxyethylstärke und Ethylstärke, genannt. Die Erzeugnisse nach der US 5 279 313 zeigen keine ausreichende Viskosität, Lagerstabilität und Gebrauchseigenschaften.

Die JP *63-62535* beschreibt die Herstellung eines stabilisierenden Emulgators, zu dessen Herstellung Lecithin und Polyglycerol in eine Lösung eines Stärkehydrolysats emulgiert werden und modifizierte Stärke und Xanthangummi zugesetzt werden. Die modifizierte Stärke ist beispielsweise Hydroxypropyldistärkephosphat, Hydroxypropylstärke oder Distärkephosphat. Der erhaltene Emulgator soll als Ersatz für Gummiarabicum geeignet sein. Als Einsatzgebiete werden Würzmittel, emulgierte Gewürze, Aromen, Backwaren, Molkereiprodukte, Arzneimittel genannt. Die JP 63-62535 enthält keinen Hinweis auf einen möglichen Einsatz in kosmetischen oder Reinigungsmittelzusammensetzungen. Die in den Beispielen verwendeten Stärken sind Kochstärken.

Die US 4 059 458 betrifft Öl-in-Wasser-Emulsionen zur Verwendung in Nahrungsmitteln, Arzneimitteln und Kosmetika, die einen Ester einer Stärke mit mindestens einer aliphatischen C₂-C₄-Carbonsäure enthält. Der Ester weist einen Substitutionsgrad von 0,05 bis 1,0 auf und kann gegebenenfalls modifiziert, oxidiert, vernetzt oder vorgelatiniert sein. Die Stärkeester sind, insbesondere in Formulierungen mit saurem pH-Wert, nicht hydrolysestabil. Dies führt zu einem unerwünschten Viskositätsabfall, zu Phasentrennung und einem unangenehmen Geruch durch freie Carbonsäuren.

Die WO 92/06778 offenbart Zusammensetzungen, die einen C₁₂-C₂₂-Alkohol, ein Alkylpolyosid und gegebenenfalls Polyosid enthalten. Das Alkylpolyosid kann u.a. Glucose, Saccharose, Maltose, Lactose, Cellobiose, sowie Stärke umfassen. Die Zusammensetzungen sind zur Anwendung in Kosmetika und Arzneimitteln vorgesehen.

Die WO 93/04185 beschreibt die enzymatische stereospezifische Herstellung von alpha-Glucosiden aus Stärke, Maltodextrin oder Maltose. Die alpha-Glucoside können in Gegenwart einer Lipase mit einer Fettsäure weiter umgesetzt werden, wobei alpha-Glucosidester erhalten werden, die als Detergentien, oberflächenaktive Mittel und Emulgatoren in kosmetischen oder pharmazeutischen Zusammensetzungen geeignet sind. Die Stärke dient hier nur als Reservoir für Glucoseeinheiten und wird zu niedermolekularen Einheiten abgebaut.

Die DD 249 912 offenbart Stärkederivate, die durch Verethern einer Stärke mit einer halogenierten Fettsäure und anschließendes Erwärmen auf 80 bis 130°C hergestellt werden, wobei intermolekulare Esterbrücken gebildet werden. Die Stärkederivate sind als Emulsionsstabilisatoren in der pharmazeutischen, kosmetischen und Nahrungsmittelindustrie geeignet. Diese Produkte sind zum Aufbau einer erhöhten Viskosität nicht geeignet.

Die JP 56-147622 beschreibt eine Emulgatorzusammensetzung, die den Monoester einer Fettsäure mit einem dreiwertigen Alkohol sowie Galactomannan, Glucomannan und/oder Stärke enthält.

Die JP 55-127308 beschreibt schwach saure emulgierte Kosmetika, die ein ölartiges kosmetisches Material, kationische Cellulose oder kationische Stärke mit einem bestimmten N-Gehalt, Triethanolamin oder Triisopropanolamin, höhere Fettsäuren und Wasser enthalten.

Die bekannten stärkehaltigen Kosmetika bzw. Reinigungsmittel weisen zahlreiche Nachteile auf. Native Stärke ist ein in kaltem Wasser unlösliches Biopolymer. Bei der Verwendung nativer Stärke ist daher bei der Herstellung der Mittel zum Aufschluß der Stärke ein Erwärmen über einen längeren Zeitraum notwendig. Beim Einarbeiten der Stärke in Wasser bzw. eine wäßrige Grundlage kann es überdies zur Klumpenbildung kommen. Neben diesen Nachteilen verfahrenstechnischer Art weisen die bekannten stärkehaltigen Produkte Nachteile bei der Anwendung auf und zeigen z.B. ein unangenehmes klebriges Gefühl auf der Haut oder ungelöste Partikel, die ein unangenehmes rauhes Gefühl vermitteln. Außerdem können Probleme bei der Lagerstabilität auftreten, die sich in einer Phasentrennung, Retrogradation der eingesetzten Stärke oder mangelnder Geruchsstabilität äußern können.

Der Erfindung lag daher die Aufgabe zugrunde, Mittel auf Stärkebasis bereitzustellen, die die geschilderten Nachteile nicht aufweisen.

Erfindungsgemäß wird diese Aufgabe durch ein Mittel zur Reinigung oder Pflege von Haut, Zähnen oder Haar oder zur Reinigung von glatten Oberflächen gelöst, das eine wäßrige Phase aufweist, die eine vorverkleisterte vernetzte Stärke, ausgewählt aus einer C₂-C₅-Hydroxyalkylstärke und einer C₂-C₁₈-Acylstärke, enthält.

Die erfindungsgemäß zu verwendende Stärke muß vernetzt sein. Eine Vernetzung der Stärkeketten läßt sich durch geeignete Vernetzungsmittel, d.h. bifunktionelle Verbindungen, erreichen. Eine bevorzugte Vernetzungsart ist die Phosphorylierung, bei der Stärke mit Phosphoroxychlorid, Phosphorpentoxid und/oder Natriumtrimetaphosphat umgesetzt wird. Hierbei werden zwei Stärkeketten durch eine anionische P-O-Gruppe vernetzt. Der anionische Charakter der Vernetzungsstellen unterstützt die emulsionsstabilisierende Wirkung der erfindungsgemäß zu verwendenden Stärke. Eine weitere bevorzugte Vernetzungsart ist die Vernetzung mittels C₄-C₁₈-Alkan- oder -Alkendicarbonsäuren, vorzugsweise C₄-C₈-Alkandicarbonsäuren, insbesondere Adipinsäure. Die Alkan- oder Alkendicarbonsäure verknüpft zwei Stärkeketten über Esterbindungen. Sie kann geradkettig oder verzweigt sein. Diese Derivate werden z.B. durch Umsetzung von Stärke mit den gemischten Anhydriden von Dicarbonsäure und Essigsäure erhalten. Bezogen auf Trockenstärke werden im allgemeinen weniger als 0,1 Gew.-%, üblicherweise etwa 0,6 Gew.-% Vernetzungsmittel verwendet.

Die Art der Modifizierung der erfindungsgemäß zu verwendenden Stärke ist kritisch. In einer Variante ist die Stärke eine C₂-C₅-Hydroxyalkylstärke. Es wird angenommen, daß die Ausbildung einer Hydroxylgruppe, die über eine Alkylgruppe mit 2 bis 5 Kohlenstoffatomen an das Stärkegerüst gebunden ist, ein geeignetes hydrophil-lipophiles Verhalten der Stärke bedingt. Die Stellung der Hydroxylgruppe in der Alkylgruppe ist nicht kritisch, diese kann in α- bis ω-Stellung sitzen. Der Substitutionsgrad der Hydroxyalkylierung beträgt vorzugsweise etwa 0,08 bis 0,3. Der Substitutionsgrad ist die Zahl der pro Anhydroglucoseeinheit durchschnittlichen Zahl an substituierten OH-Gruppen der Stärkemoleküle. Die Hydroxyalkylierung einer nativen Stärke kann durch Umsetzen einer nativen Stärke mit Alkenoxiden der geeigneten Kohlenstoffzahl erreicht werden. Besonders bevorzugt sind hydroxyethylierte und/oder hydroxypropylierte Stärken, die durch Umsetzung von Stärken mit Ethylenoxid bzw. Propylenoxid erhalten werden. Eine erfindungsgemäß zu verwendende Stärke kann pro Alkylgruppe auch mehr als eine Hydroxylgruppe enthalten.

In einer anderen Variante ist die Stärke eine C₂-C₁₈-Acylstärke. Dieser Fall liegt regelmäßig vor, wenn die oben erläuterte erfindungswesentliche Vernetzung über C₄-C₁₈-Dicarbonsäuren bewirkt wurde. Ein hierdurch erhaltenes Distärke-C₄-C₁₈-alkan- oder-alkenoat kann im Hinblick auf ein geeignetes hydrophil-lipophiles Verhalten mit einem Substitutionsgrad von 0 bis 0,8, insbesondere 0 bis 0,5 zusätzlich acyliert sein. Die Acylierung erfolgt allgemein insbesondere durch Umsetzung mit Säureanhydriden der allgemeinen Formel (R-C(O))₂O, wobei R eine Alkylgruppe, wie Methyl oder Ethyl ist, mit Bernsteinsäure- oder Maleinsäureanhydrid oder deren alkylierten Derivaten.

Ein besonders bevorzugtes Stärkederivat für die Zwecke der Erfindung ist ein Hydroxypropyldistärkephosphat sowie acetyliertes Distärkeadipat.

Das Stärkeausgangsmaterial kann einer beliebigen Pflanzenquelle entstammen, es ist allerdings bevorzugt, daß die Stärke einen Amylopektingehalt von mindestens etwa 70 Gew.-%, vorzugsweise etwa 85 Gew.-%, insbesondere etwa 90 Gew.-% aufweist. Bevorzugte Stärken stammen aus Wachsmais.

Ein entscheidendes Merkmal der Erfindung besteht darin, daß das erfindungsgemäß zu verwendende Stärkederivat vorverkleistert ist. Neben dem Begriff "vorverkleisterte Stärke" sind im Stand der Technik auch die Begriffe "vorgelatinierte Stärke" und "kaltquellende Stärke" gebräuchlich. Die Bezeichnung "vorverkleisterte" oder "gelatinierte" Stärke bezieht sich auf gequollene Stärkepartikel, die ihre Doppelbrechungskreuze in polarisiertem Licht verloren haben. Vorverkleisterte Stärken bzw. Stärkederivate sind ohne Kochen bereits in kaltem Wasser löslich. "Löslich" bedeutet in diesem Zusammenhang nicht notwendig die Bildung einer echten molekularen Lösung, vielmehr wird meistens eine kolloidale Dispersion erhalten. Das erfindungsgemäß zu verwendende Stärkederivat ist vorzugsweise vollständig vorverkleistert.

Die üblicherweise zur Herstellung derartiger Stärken angewendeten Verfahren sind u.a. Trommeltrocknung, Extrusion und Sprühtrocknung.

Die Trommeltrocknung umfaßt das gleichzeitige Kochen und Trocknen einer sehr hochviskosen, halbfesten Stärkepaste auf erhitzten Trommeln. Die getrockneten Filme werden von der Trommel mit einem Metallmesser abgestreift und dann gemahlen. Dieses Verfahren kann bis zu einem sehr hohen Feststoffgehalt durchgeführt werden.

Man kann auch die Extrusion zum gleichzeitigen Kochen und Trocknen von Stärken einsetzen (vgl. die US 3 137 592). Diese Methode bedient sich der physikalischen Bearbeitung einer Stärke/Wasser-Mischung bei erhöhten Temperaturen und Drucken, was die Gelatinierung der Stärke bewirkt, gefolgt von der Expansion nach Austritt aus der Düse unter schlagartiger Verdampfung des Wassers.

Die Verwendung eines vorverkleisterten Stärkederivates gestattet die Herstellung des erfindungsgemäßen Mittels bei Raumtemperatur, bzw. bei einer Temperatur, die gegenüber den Herstellungsbedingungen bekannter stärkehaltiger Zusammensetzungen erheblich tiefer liegt. Man hat überraschenderweise gefunden, daß mit einer Kochstärke (d.h. nicht vorverkleisterten Stärke), die in gleicher Weise wie eine erfindungsgemäß zu verwendende Stärke modifiziert ist, nicht die gewünschten Vorteile hinsichtlich Rheologie, Hautgefühl und Emulsionsstabilität erhalten werden, selbst wenn die wäßrige Phase nach Zugabe der Kochstärke 15 Minuten lang auf eine Temperatur oberhalb der Gelatinierungstemperatur der Kochstärke erhitzt wird.

Vorzugsweise ist das vorverkleisterte Stärkederivat durch Sprühtrocknung hergestellt. Bei anderen Trocknungsverfahren, z.B. der Trommeltrocknung, kann es zur Bildung von Stärkekrusten kommen, die eine schlechtere Löslichkeit aufweisen. Dies führt zu ungelösten Partikeln im erfindungsgemäßen Mittel, die unangenehmes sandiges Gefühl auf der Haut hervorrufen können.

Vorzugsweise enthält das erfindungsgemäß zu verwendende Stärkederivat weitgehend intakte Stärkekörner. Es hat sich gezeigt, daß die wäßrigen Dispersionen vorverkleisterter Stärkederivate mit weitgehend intakter Kornstruktur eine gleichmäßigere glatte Textur aufweisen als wäßrige Dispersionen von Stärken ohne Kornstruktur, die z.B. durch Trocknung von Stärkelösungen erhalten werden und deren Dispersionen sich leicht grießig anfühlen. Bei vorverkleisteten Stärken mit intakter Kornstruktur ist die native Binnenstruktur von Wasserstoffbrückenbindungen zerstört, die äußere Form bleibt jedoch erhalten.

Ein Verfahren zur Herstellung besonders geeigneter sprühgetrockneter vorverkleisteter Stärken bzw. Stärkederivate wird in der US 4 280 851 beschrieben. Eine zur Durchführung des Verfahrens geeignete Vorrichtung wird in der US 4 600 472 beschrieben. Bei diesem Verfahren wird eine Mischung der (des) granularen Stärke(derivates) im zerstäubten Zustand gekocht oder gelatiniert. Die zu kochende Stärke wird durch eine Zerstäubungsöffnung in eine Düsenanordnung gespritzt, um ein relativ fein zerteiltes Sprühgut zu bilden. Ausserdem wird ein Heizmedium durch eine Öffnung in der Düsenanordnung in das Sprühgut eingespritzt, um so die Stärke auf eine zur Gelatinierung erforderliche Temperatur zu erhitzen. Eine geschlossene Kammer umgibt die Einspritzöffnungen für das Zerstäubungs- und Heizmedium und definiert eine Entlüftungsöffnung, die so angeordnet ist, daß das erhitzte Stärkesprühgut die Kammer verlassen kann. Die Anordnung ist so geschaffen, daß die während der Passage des Stärkesprühgutes durch die Kammer, d.h. von der Zerstäubungsöffnung bis zur Entlüftungsöffnung, verstrichene Zeit die Gelätinierungszeit der Stärke definiert. Die resultierende sprühgetrocknete, vorgelatinierte Stärke umfaßt gleichmäßig gelatinierte Stärkekörner in Form eingedellter Kügelchen, wobei ein Hauptanteil der Körnchen ganz und unzerbrochen ist und nach Hydration quillt. Düsen, die zur Herstellung dieser Stärken verwendbar sind, werden auch in der US 4 610 760 beschrieben.

Zur Herstellung geeigneter vorverkleisterter Stärken bzw. -derivate ist auch das Verfahren der US 5 149 799 geeignet. Bei diesem Verfahren wird Stärke mittels eines einzigen Zerstäubungsschrittes in Gegenwart eines wäßrigen Mediums gleichzeitig zerstäubt und gekocht. Der Zerstäubungsschritt wird in einer Vorrichtung durchgeführt, die eine Zweistoff-Sprühtrocknungsdüse mit Innenmischung aufweist, welche an eine Vorrichtung zum Trocknen der gekochten zerstäubten Stärke gekoppelt ist.

Sprühgetrocknete, vorgelatinierte Stärken bzw. -derivate mit geeigneten Eigenschaften können auch durch ein kontinuierliches gekoppeltes Dampfstrahlkoch- und Sprühtrocknungsverfahren hergestellt werden. Eine Stärkeaufschlämmung wird bei 138° bis 160°C in einem Jet-Cooker unter direktem Wasserdampfeintrag gelatiniert. Die Ströme von Stärkeaufschlämmung und Wasserdampf werden in einer Kochkammer gemischt. Der Auslaß der Kochkammer ist an eine pneumatische Sprühdüse oder eine Hochdruck-Düse angeschlossen, die sich in einem üblichen Sprühtrockner befindet. Die strahlgekochte Stärke wird noch bei erhöhter Temperatur und unter erhöhtem Druck in die Sprühdüse gerichtet und kann mit Kaltluft, Heißluft oder vorzugsweise Wasserdampf zerstäubt werden. Nach dem Zerstäuben der heißen, strahlgekochten Stärkelösung wird diese in der gleichen Weise wie übliche sprühgetrocknete Stärken gehandhabt. Das Trocknungsverfahren ist ausreichend schnell, eine Retrogradation der Stärkemoleküle zu verhindern, während die Tröpfchen abkühlen und trocknen. Die sprühgetrocknete Stärke ist ein amorpher Feststoff (d.h. er ist im wesentlichen nicht kristallin) und leicht in Wasser löslich bzw. kolloidal dispergierbar.

Das erfindungsgemäße Mittel kann in beliebiger Form, z.B. als Lösung, Emulsion, Suspension oder Gel oder Schaum, vorliegen. Es kann aber auch als trockene pulverförmige Zusammensetzung vorliegen, die bei der Anwendung in einem wäßrigen Medium aufgenommen wird. Das Mittel weist im allgemeinen vorzugsweise etwa 5 bis 98 Gew.-%, insbesondere etwa 50 bis 90 Gew.-% wäßrige Phase auf. Die wäßrige Phase enthält vorzugsweise etwa 0,1 bis 20 Gew.-%, insbesondere 0,3 bis 12 Gew.-% und besonders bevorzugt etwa 0,5 bis 7 Gew.-% Stärkederivat mit den oben angegebenen Merkmalen. Das erfindungsgemäß zu verwendende Stärkederivat kann in Verbindung mit anderen Stärken, wie nativen Stärken, modifizierten Stärken usw. eingesetzt werden.

Das erfindungsgemäß zu verwendende Stärkederivat erfüllt im erfindungsgemäßen Mittel verschiedene Funktionen. Sie wirkt 1) als Stabilitätsverbesserer, 2) als Viskositätsregulator, 3) als (Co)-Emulgator, 4) als Mittel zur Verbesserung des Hautgefühls und 5) als Mittel zur Verbesserung der Frisiereigenschaften.

Wäßrige Dispersionen derartiger Stärkederivate sind durch eine Vielfalt fettähnlicher Texturen gekennzeichnet, die von ölig über cremig bis wachsartig reichen. Diese Stärkederivate können so ausgewählt werden, daß in wäßrigen Dispersionen Stärkegele hoher Festigkeit oder wärmereversible Stärkegele erhalten werden. Ein wärmereversibles Stärkegel ist ein solches, das beim Erhitzen schmilzt und sich nach dem Abkühlen erneut bildet. Aus unmodifizierten Stärken hergestellte Gele sind nicht wärmereversibel. Mit den erfindungsgemäß zu verwendenden Stärken können daher Öl oder Fett ganz oder teilweise ersetzt werden und beispielsweise ölfreie Lotionen formuliert werden, die den Eigenschaften von Öl-in-Wasser-Emulsionen weitgehend ähneln.

Die erfindungsgemäß zu verwendenden Stärkederivate weisen Anwendungseigenschaften auf, die vom dermatologischen Standpunkt her wünschenswert sind. So erhöhen sie das Wasserrückhaltevermögen der Haut und machen die Haut glatt und geschmeidig. Pflegeprodukte, die die erfindungsgemäß zu verwendenden Stärkederivate enthalten, lassen sich ausgezeichnet auf der Haut verstreichen und hinterlassen kein klebriges Gefühl.

Die erfindungsgemäß zu verwendenden Stärkederivate weisen einen stabilisierenden und ölbindenden Effekt auf. Sie verhindern ein Absetzen fester Bestandteile sowie die Phasentrennung flüssiger Bestandteile. Die haben außerdem einen viskositätserhöhenden Effekt und verleihen dem erfindungsgemäßen Mittel einen ansprechenden Glanz. Die erfindungsgemäß zu verwendenden Stärkederivate weisen daneben eine gewisse Schutzkolloidwirkung auf und verhindern somit das Zusammenfließen emulgierter Tröpfchen einer hydrophoben Phase. Aufgrund dieser Wirkung als (Co)-Emulgator kann die Menge oberflächenaktiver Emulgatoren bei erfindungsgemäßen Pflegemitteln im Vergleich zu herkömmlichen Pflegemitteln herabgesetzt werden. Im Einzelfall kann auf oberflächenaktive Emulgatoren ganz verzichtet werden. Dies bietet gegenüber anderen Produkten Vorteile bei Kosmetika, für die "natürliche" und "hyperallergene" Eigenschaften herausgestellt werden. Die erfindungsgemäß zu verwendenden Stärkederivate weisen außerdem substantive Eigenschaften auf, d.h. sie können z.B. auf das menschliche Haar aufziehen und dieses leichter kämmbar und geschmeidiger machen.

Die ölbindenden Eigenschaften der erfindungsgemäß zu verwendenden Stärkederivate führen zu einem verbesserten Transport öllöslicher Wirkstoffe erfindungsgemäßer Pflegemittel auf die Hautoberfläche.

In einer bevorzugten Ausführungsform enthält das erfindungsgemäße Mittel mindestens etwa 0,3, vorzugsweise etwa 7 bis 25, in Einzelfällen bis zu 70 Gew-% oberflächenaktive Substanzen. Diese Mittel sind insbesondere Mittel, bei denen eine Reinigungswirkung im Vordergrund steht, z.B. ein Shampoo, Duschgel, Schaumbad, eine flüssige Seife bzw. ein Handgeschirrspülmittel, oder ein Haarnachbehandlungsmittel. Diese Mittel werden im folgenden kollektiv als WAS/Stärke-Kombinationen bezeichnet (WAS = waschaktive Substanzen).

Die grenzflächenaktiven Substanzen sind vorzugsweise anionische, amphotere und/oder nichtionische Tenside. Als anionische Tenside können die Alkalimetallsalze von Alkylsulfonaten oder -sulfaten mit 8 bis 22 Kohlenstoffatomen in der Alkylkette genannt werden. Die Natrium-, Ammonium-, Kalium- oder Triethanolammoniumalkylsulfate sind bevorzugt, insbesondere diejenigen, die durch Sulfatierung höherer Alkohole (8 bis 18 Kohlenstoffatome) erhalten sind, ferner Natriumsalze von Kokosnußölfettsäuremonoglyceridsulfaten oder -sulfonaten, Natrium- oder Kaliumsalze von Schwefelsäureestem der Addukte von 1 bis 12 Mol Ethylenoxid an höhere Fettalkohole (z.B. Talk- oder Kokosnußölalkohole), Natrium- oder Kaliumsalze von Alkylphenol-Ethylenoxid-Ethersulfaten mit 1 bis 10 Ethylenoxideinheiten pro Molekül, in denen die Alkylreste 8 bis 12 Kohlenstoffatome enthalten, Natriumalkylglycerylethersulfonate, Reaktionsprodukte von C₁₀-C₂₂-Fettsäuren, verestert mit Isethionsäure, Natriumsalz, wasserlösliche Salze von Kondensationsprodukten von Fettsäuren mit Sarcosin. Weitere anionische Tenside sind Sulfoacetate und Sulfosuccinate.

Als zwitterionische Tenside können diejenigen genannt werden, die als Derivate von aliphatischen quarternären Ammonium-, Phosphonium- und Sulfoniumverbindungen beschrieben werden können, bei denen die aliphatischen Reste geradkettig oder verzweigt sein können und einer der aliphatischen Subsituenten 8 bis 18 Kohlenstoffatome enthält und einer eine anionische Gruppe, z.B. eine Carboxy-, Sulfonat-, Sulfat-, Phosphat- oder Phosphonaigruppe, enthält. Beispiele hierfür sind 4-[N,N-Di(2-hydroxyethyl)-N-octadecylammonio]-butan-1-carboxylat, 5-[S-3-Hydroxypropyl-S-hexadecylsulfonio]-3-hydroxypentan-1-sulfat, 3-[P,P-Diethyl-P-3,6,9-trioxatetradioxylphosphonio]-2-hydroxypropan-1-phosphat, 3-[N,N-Dipropyl-N-3-dodecoxy-2-hydroxypropylammonio]-propan-1-phosphonat, 3-(N,N-Dimethyl-N-hexadecylammonio)-propan-1-sulfonat, 3-(N,N-Dimethyl-N-hexadecylammonio)-2-hydroxypropan-1-sulfonat, 4-[N,N-Di(2-hydroxyethyl)-N-(2-hydroxydodecyl)-ammonio]-butan-1-carboxylat, 3-[S-Ethyl-S-(3-dodecoxy-2-hydroxypropyl)-sulfonio]-propan-1-phosphat, 3-[P,P-Dimethyl-P-dodecylphosphonio]-propan-1-phosphonat und 5-[N,N-Di(3-hydroxypropyl)-N-hexadecylammonio]-2-hydroxypentan-1-sulfat. Betaine sind für die vorliegende Erfindung ebenfalls geeignet. Geeignete Betaine sind u.a. die höheren Alkylbetaine, z.B. Kokosdimethylcarboxymethylbetain, Lauryldimethylcarboxymethylbetain, Lauryldimethylalphacarboxyethylbetain, Cetyldimethylcarboxymethylbetain, Lauryl-bis(2-hydroxyethyl)-carboxymethylbetain, Stearylbis(2-hydroxypropyl)-carboxymethylbetain, Oleyldimethyl-gamma-carboxypropylbetain, Lauryl-bis(2-hydroxypropyl)-alpha-carboxyethylbetain usw., Sulfobetaine, wie Kokosdimethylsulfopropylbetain, Stearyldimethylsulfopropylbetain, Lauryldimethylsulfoethylbetain, Lauryl-bis(2-hydroxyethyl)-sulfopropylbetain und dergleichen. Amidobetaine und Amidosulfobetaine, bei denen ein RCONH(CH2)3-Rest an das Stickstoffatom des Betains geknüpft ist, sind ebenfalls geeignet.

Nichtionische Tenside, die vorzugsweise in Kombination mit einem anionischen oder amphoteren Tensid verwendet werden, können grob als Verbindungen definiert werden, die durch Kondensation eines Alkylenoxids an eine hydrophobe organische Verbindung hergestellt werden. Beispiele bevorzugter Klassen nichtionischer Tenside sind Polyethylenoxidkondensate von Alkylphenolen, z.B. die Kondensationsprodukte von Alkylphenolen mit 6 bis 12 Kohlenstoffatomen in der Alkylgruppe mit 10 bis 60 Mol Ethylenoxid, Kondensationsprodukte von Ethylenoxid mit Propylenoxid-Ethylendiamin-Umsetzungsprodukten, Kondensationsprodukte von aliphatischen Alkoholen mit 8 bis 18 Kohlenstoffatomen mit Ethylenoxid, langkettige tertiäre Aminoxide, langkettige tertiäre Phosphinoxide, langkettige Dialkylsulfoxide. Der Begriff "langkettig" bedeutet, daß im Molekül mindestens eine lange hydrophobe Kette vorliegt, die einen Alkyl-, Alkenyl-, Hydroxyalkyl- oder Ketoalkylrest mit 8 bis 20 Kohlenstoffatomen enthält. Weitere Beispiele für nichtionische Tenside sind Alkyl- und Alkenyloligoglycoside, wie C12/C14-Kokosalkyloligoglucosid.

Für Haarpflegemittel und Haarkonditionierungsmittel werden üblicherweise kationische Verbindungen, vorzugsweise quaternäre Ammoniumverbindungen, wie Cetyltrimethylammoniumchlorid, oder diquaternäre Polydimethylsiloxane, verwendet. Das erfindungsgemäß einzusetzende Stärkederivat weist den Vorteil auf, daß es mit den kationischen Verbindungen kompatibel ist, d.h. auch in Gegenwart der kationischen Verbindung dispergiert bleibt und seine Viskosität bewahrt.

Das Mittel weist vorzugsweise einen pH-Wert zwischen etwa 2,5 und 12, insbesondere zwischen etwa 4 und 9, auf. Ein gewünschter pH-Wert wird durch Zusatz geeigneter pH-Regulatoren, wie Citronensäure, Milchsäure, Phosphorsäure, Salzsäure, Natriumhydroxid, Kaliumhydroxid oder Triethanolamin erreicht. Bei Einsatz von Stärkeestern ist ein stark saurer pH-Wert möglichst zu vermeiden, da sonst eine Hydrolyse der Esterbindungen erfolgt.

In einer weiteren bevorzugten Ausführungsform enthält das erfindungsgemäße Mittel mindestens etwa 1 Gew.-%, insbesondere etwa 5 bis 25 Gew.-%, in Einzelfällen bis zu etwa 70 Gew.-%, hydrophobe Phase. Die hydrophobe Phase ist vorzugsweise fein dispergiert. Die hydrophobe Phase kann der hydrophoben Phase herkömmlicher Kosmetika auf Öl-in-Wasser-Basis entsprechen und enthält beispielsweise flüssige oder feste Fettsäuretriglyceride, Kohlenwasserstoffe, Alkylether, Alkylethoxylate, Alkylpropoxylate, Alkylbutoxylate, Fettsäuremono- oder -diester, Silikone und/oder langkettige Alkohole. Diese Mittel werden im folgenden kollektiv als Mittel vom Emulsionstyp bezeichnet.

Um die hydrophobe Phase in feiner Dispersion zu halten, enthalten die erfindungsgemäßen Mittel vom Emulsionstyp vorzugsweise einen oder mehrere zusätzliche Emulgatoren, insbesondere einen Teilester eines mehrwertigen Alkohols, ein Ethyoxylat, Propoxylat und/oder Butoxylat eines HLB-Wertes von etwa 4 bis 16 und/oder ionische Emulgatoren, wie Citrate oder Tartrate von Monoglyceriden. Geeignet sind auch Kombinationen von Glycerin-mono- oder -distearat mit Fettalkoholsulfaten oder Alkalisalzen von Fruchtsäureestern der Glycerin-mono- oder -difettsäureester. Der Emulgator liegt vorzugsweise in einer Menge von 0,1 bis 10 Gew.-%, insbesondere etwa 0,5 bis 5 Gew.-% vor. Das erfindungsgemäße Mittel kann in dieser Ausführungsform als Creme, Lotion oder Milch vorliegen. Je nach der Beschaffenheit und Menge der wäßrigen bzw. hydrophoben Phase können in den Mitteln vom Emulsionstyp verschiedenartige Typen von Emulsionen vorliegen, z.B. binäre Öl-in-Wasser- oder Wasser-in-Öl-Systeme oder multiple Systeme, wie Wasser-in-Öl-in-Wasser- oder Öl-in-Wasser-in-Ol-Systeme.

In einer weiteren bevorzugten Ausführungsform liegt das erfindungsgemäße Mittel als hochviskoses Gel vor. Das erfindungsgemäße Gel enthält vorzugsweise einen oder mehrere ein- oder mehrwertige Alkohole. Hierbei sind insbesondere Glycerin und/oder Ethanol geeignet. Der Alkohol liegt im erfindungsgemäßen Gel vorzugsweise in einer Menge von etwa 5 bis 25 Gew.-% vor. Eine Menge von 25 Gew.-% sollte nicht überschritten werden, da sonst eine Ausfällung der Stärke erfolgen kann. Das Gel kann Mittel enthalten, die auf der Haut eine erfrischende und abkühlende Wirkung verleihen, wie z.B. Menthol oder Menthyllactat.

Eine weitere bevorzugte Ausführungsform der Erfindung ist ein Haarfärbe- oder Haarbleichmittel. Diese Mittel sind durch einen Gehalt an Farbmittel bzw. Oxidationsmittel gekennzeichnet. Als Oxidationsmittel sind im allgemeinen Perverbindungen, wie Peroxide, z.B. Wasserstoffperoxid, oder Persulfate geeignet. Das Haarfärbe- bzw.-bleichmittel kann als Einkomponentenmittel oder vorzugsweise als Zweikomponentenmittel vorliegen. Die zwei Komponenten des Mittels werden vom Anwender vor der Anwendung vereinigt und auf das Haar aufgetragen. Wenn das Mittel als zwei Komponenten vorliegt, ist es bevorzugt, daß eine Komponente pulverförmig und die andere Komponente flüssig ist. Die pulverförmige Komponente enthält das erfindungsgemäß zu verwendende Stärkederivat, die flüssige Komponente enthält eine wäßrige Phase. Haarfärbe- bzw. Haarbleichmittel weisen im allgemeinen einen hohen pH-Wert auf. Überraschenderweise sind die erfindungsgemäß zu verwendenden Stärkederivate auch bei den erhöhten pH-Werten dieser Produkte stabil. Die durch das erfindungsgemäß zu verwendende Stärkederivat hervorgerufene Viskositätserhöhung bewirkt, daß das Produkt während der Anwendung im Haar verbleibt und eine gewünschte Einwirkdauer erreicht wird. Die erfindungsgemäß zu verwendende Stärke läßt sich leicht und klumpenfrei im wäßrigen Medium dispergieren. Herkömmlicherweise in Haarbehandlungsmitteln eingesetzte Polysaccharidverdickungsmittel, wie Hydroxyethylcellulose und Xanthangummi sind üblicherweise schwierig zu dispergieren, da sie leicht klumpen und Fischaugen bilden.

Für sämtliche Ausführungsformen der vorliegenden Erfindung gilt, daß das erfindungsgemäße Mittel je nach Anwendungszweck Zusatzstoffe in Form von Konservierungsmitteln, Duftstoffen, Aromastoffen, Lichtschutzmitteln, Antioxidantien, Vitaminen, pharmazeutischen Wirkstoffen, Füllstoffen, Sequestriermitteln, Färbemitteln, Bräunungsmitteln, zusätzlichen Verdickungsmitteln, anorganischen Salzen, pH-Regulatoren und/oder Perlglanzmitteln enthalten kann.

Das erfindungsgemäße Mittel weist zahlreiche Vorteile auf. So handelt es sich bei den Stärkerohstoffen um einen nachwachsenden Rohstoff, was dem Konsumverhalten einer steigenden Zahl von Verbrauchern entgegenkommt. Die Stärkerohstoffe werden bereits seit langem in Lebensmitteln eingesetzt und können als unbedenklich gelten. Im Vergleich zu herkömmlicherweise eingesetzten Carbomeren enthalten sie keine toxischen Monomere und keine Lösungsmittel. Sie enthalten außerdem keine Pestizide, die z.B. in Pflanzengummis enthalten sein können.

Die Erfindung wird nun durch die folgenden Beispiele näher veranschaulicht. Die pH-Werte wurden elektrometrisch im unverdünnten Produkt gemessen. Die Viskositätswerte wurden mit einem Viskositätsmeßgerät der Firma Haake Typ RV 20-Rotovisko mit den bei den Beispielen erwähnten Meßeinrichtungen nach einer Minute Scherzeit gemäß DIN 53019 gemessen. Die Ansätze wurden im 1 kg-Maßstab mit einem Flügelrührer gefertigt. Die Homogenisation erfolgte mit einem Homogenisiergerät Typ S 100 SL 90 der Firma Fema AG bei Stufe 8 und einer Durchlaufzeit von 100 ml/min.

In den Beispielen werden folgende Bezeichnungen und Handelsnamen verwendet.

| Handelsname | Hersteller | INCI Name |
|---|---|---|
| Dehyquart A | Henkel KGaA | Cetrimonium Chloride |
| Jaguar C-162 | Rhone-Poulenc | Hydroxypropyl Guar Hydroxypropyltrimonium Chloride |
| Lanette 14 | Henkel KGaA | Myristyl Alcohol |
| Tegopearl N 100 | TH.Goldschmidt AG | Glycol Distearate, Steareth-4 |
| Texapon N 70 | Henkel KGaA | Sodium Laureth Sulfate |
| Plantaren 1200 | Henkel KGaA | Lauryl Glucoside |
| Tego Betain F | TH.Goldschmidt AG | Cocamidopropyl Betaine |
| Elfan NS 242 A | Akzo Nobel | Sodium Laureth Sulfate |
| Tego Betain F 50 | TH.Goldschmidt AG | Cocamidopropyl Betaine |
| Axol C62 | TH.Goldschmidt AG | Glyceryl Stearate Citrate |
| Nipagin | Nipa Laboratorien GmbH | Methylparaben |
| Nipasol | Nipa Laboratorien GmbH | Propylparaben |
| Prisorine 3700 | Unichema International | Polyglyceryl-3 Diisostearate |
| Tegin 90 | TH.Goldschmidt AG | Glyceryl Stearate |
| Miglyol 812 | Hüls AG | Caprylic/Capric Triglyceride |
| Sucro-Ester WE 15 | Gattefosse GmbH | Sucrose Palmitate |
| Cetiol 868 | Henkel KGaA | Octyl Stearate |
| Phenonip | Nipa Laboratorien GmbH | Phenoxyethanol, Methylparaben, Butylparaben, Ethylparaben, Propylparaben |
| Parsol MCX | Givaudan-Roure | Octyl Methoxycinnamate |
| Uvinul MS 40 | BASF | Benzophenone-4 |
| Natrium- Hyaluronat | ROVI GmbH | Sodium Hyaluronate |
| Vitamin A-Palmitat | BASF AG | Retinyl Palmitate |
| Vitamin E Acetat | BASF AG | Tocopheryl Acetate |
| Ronoxan A | Hoffmann La Roche AG | Ascorbylpalmitat, D.L- alpha -Tocopherol, Lecithin (deutsche Bezeichnungen) |
| Tego Care 215 | TH.Goldschmidt AG | Ceteareth-15, Glyceryl Stearate |
| Tego Care 450 | TH.Goldschmidt AG | Polyglyceryl-3 Methylglucose Distearate |
| Protegin | TH.Goldschmidt AG | Mineral Oil, Perolatum, Ozokerite, Glyceryl Oleate, Lanolin Alcohol |
| Neo PCL W/O E 2/066255 | Dragoco Gerberding & Co.AG | Cetearyl Octanoate, Ceresin, Lanolin, Sorbitan Sesquioleate, Stearyl Heptanoate. Paraffinium liquidum, Trihydroxystearin, BHT |
| Hydroviton Feuchthaltefaktor | Dragoco Gerberding & Co.AG | Aqua. Sodium Lactate, Lactic Acid, Glycerin, Serine, Sorbitol, TEA Lactate, Triethanolamine, Urea: Sodium Chloride, Lauryl Diethylenediaminoglycerine, Allantoin, Lauryl Aminopropylglycerine, Alcohol |
| Texapon ALS | Henkel KGaA | Ammonium Lauryl Sulfate |
| Rewopol SBFA | Witco Surfactants GmbH | Disodium Laurethsulfosuccinate |
| Euxyl K400 | Schülke&Mayr | Methyldibromo Glutaronitril, Phenoxyethanol |
| Sident 12DS | Degussa | Silica |
| Texapon K1296 Pulver | Henkel KGaA | Sodium Lauryl Sulfate |
| Saccharin Na | Bayer AG | Sodium Saccharin |
| Cutina FS 45 | Henkel KGaA | Palmitic Acid, Stearic Acid |
| Luviskol K30 | BASF AG | PVP |
| Cyclodextrin Beta W7 | Wacker GmbH | Cyclodextrin |
| Pionier 4656 | Hansen & Rosenthal | Mineal Oil |
| Vaseline | Hansen & Rosenthal | Petrolatum |
| Titandioxid | Les Colorants Wackherr S.A. | |

### Beispiele 1 bis 3:

Diese Beispiele erläutern die Herstellung einer Haar-Spülung. Es wurde nach folgender Rezeptur verfahren:

| Rohstoff | Beispiel 1 Gew.% | Beispiel 2 Gew.-% | Beispiel 3 Gew.-% |
|---|---|---|---|
| Wasser | 87,050 | 87,050 | 89,750 |
| Dehyquart A | 3,000 | 3,000 | 3,000 |
| Jaguar C-162 | 0.500 | 0,500 | 0.500 |
| vorverkleistertes hydroxypropyliertes Distärkephosphat von Wachsmaisstärke in Form lose agglomerierter Körner | 2,700 | | |
| hydroxypropyliertes Distärkephosphat von Tapiokastärke *) | | 2,700 | |
| Lanette 14 | 3,000 | 3,000 | 3,000 |
| Tegopearl.N 100 | 3.000 | 3,000 | 3,000 |
| Parfüm | 0.400 | 0,400 | 0,400 |
| Citronensäure | 0,050 | 0.050 | 0.050 |
| Benzoesäure | 0.300 | 0,300 | 0,300 |
| Spezifikationswerte: | | | |
| pH-Wert | 5,1 | 5,2 | 5,1 |
| Viskosität MV Stufe 1 (mPas) | 5100 | 1350 | 1100 |

| | | | |
|---|---|---|---|
| *) Farinex VA 70T (Avebe/Stadex, Malmö, Schweden) | | | |

Zur Herstellung werden in das auf ca. 55°C erwärmte Wasser Dehyquart A, Benzoesäure und die Stärkederivate - Beispiel 3 ohne - eingerührt. Wenn diese Mischung homogen ist, werden Jaguar C-162 und Citronensäure eingerührt. Lanette 14 wird auf 55° C erhitzt und in den Ansatz eingerührt. Nach Abkühlen unter Rühren bis auf ca. 40°C werden Tegopearl N 100 und das Parfüm dem Ansatz zugesetzt. Nach dem Abkühlen unter Rühren bis auf ca. 25°C resultiert im Beispiel 1 eine mittelviskose perlglänzende Dispersion. Die Beispiele 2 und 3 waren zu dünnflüssig und damit nicht brauchbar.

Die Haar-Spülung gemäß Beispiel 1 wurde bei Anwendungsversuchen an normalen und geschädigten Haaren gut beurteilt. Die Beispiele 2 und 3 führten bezüglich der Verteilbarkeit auf dem nassen Haar und der Naßkämmbarkeit zu schlechten Ergebnissen.

Die Lagertests an Beispiel 1 bei -18°C, +4°C, Raumtemperatur (18°C bis 25°C), +30°C und +40°C zeigten nach 1 Monat ein hinsichtlich der Spezifikationen weitgehend unverändertes Produkt. Die Beispiele 2 und 3 zeigten nach 1 Tag Phasentrennung.

### Beispiele 4 bis 6:

Diese Beispiele erläutern die Herstellung eines Shampoos. Es wurde nach folgender Rezeptur verfahren:

| Rohstoff | Beispiel 4 Gew.-% | Beispiel 5 Gew.-% | Beispiel 6 Gew.-% |
|---|---|---|---|
| Wasser | 73,21 | 73,21 | 73.81 |
| Texapon N 70 | 11,00 | 11,00 | 11,00 |
| Plantaren 1200 | 3,50 | 3,50 | 3,50 |
| Natriumbenzoat | 0,40 | 0,40 | 0,40 |
| Kaliumsorbat | 0,20 | 0,20 | 0,20 |
| Citronensäure | 0,49 | 0,49 | 0,49 |
| Tego Betain F | 4,20 | 4,20 | 4,20 |
| Natriumchlorid 22 % Lösung in Wasser | 6,00 | 6,00 | 6,00 |
| D-Panthenol | 0,10 | 0,10 | 0,10 |
| vorverkleistertes hydroxypropyliertes Distärkephosphat von Wachsmaisstärke in Form lose agglomerierter Körner | 0,60 | | |
| hydroxypropyliertes Distärkephosphat von Kartoffelstärke *) | | 0,60 | |
| Parfüm | 0,30 | 0,30 | 0,30 |
| Spezifikationswerte: | | | |
| pH - Wert | 5.0 | 5,1 | 5,2 |
| Viskosität MV Stufe 1 (mPas) | 11100 | 7200 | 7000 |

| | | | |
|---|---|---|---|
| *) Farinex VA 70 (Avebe/Stadex, Malmö, Schweden) | | | |

In das ca. 25°C warme Wasser werden nacheinander Plantaren 1200, Natriurnbenzoat, Kaliumsorbat, Citronensäure, Tego Betain F, D-Panthenol und die Stärkederivate - Beispiel 6 ohne - eingerührt. Nachdem die Mischung homogen ist, wird die 22%ige Natriumchlorid-Lösung eingerührt. Unter starkem Rühren wird Texapon N 70 zugegeben. Wenn die Mischung homogen ist, wird das Parfüm eingerührt.

Gemäß Beispiel 4 resultiert ein leicht opaleszierendes viskoses Shampoo. Die Beispiele 5 und 6 sind deutlich niedriger viskos.

Bei der Anwendung am Haar zeigt das Shampoo nach Beispiel 4 neben einem feinblasigen Schaum eine gute Naßkämmbarkeit. Die Beispiele 5 und 6 bilden auf dem Haar einen deutlich grobblasigeren Schaum als das Beispiel 4. Ebenso ist die Naßkämmbarkeit schlechter.

Die Lagertests des Produktes gemäß Beispiel 4 bei -18°C, +4°C, Raumtemperatur (18°C bis 25°C) und + 30°C zeigten nach 1 Monat ein hinsichtlich der Spezifikationen weitgehend unverändertes Produkt.

Beispiel 5 zeigte nach 2 Tagen bei Raumtemperatur Phasentrennung.

Die Viskosität von Beispiel 4 ist tatsächlich höher als der angegebene Wert, weil der Meßbereich des verwendeten Viskosimeters überschritten war.

### Beispiele 7 und 8:

Diese Beispiele erläutern die Herstellung eines Schaumbads. Es wurde nach folgender Rezeptur verfahren:

| Rohstoff | Beispiel 7 Gew.-% | Beispiel 8 Gew.-% | Beispiel 9 Gew.-% |
|---|---|---|---|
| Elfan NS 242 A | 89,38 | 89,38 | 90,08 |
| Glycerin 87 % | 2,72 | 2,72 | 2,72 |
| Tego Betain F 50 | 3,00 | 3,00 | 3,00 |
| Plantaren 1200 | 2,00 | 2,00 | 2,00 |
| Kaliumsorbat | 0,20 | 0,20 | 0,20 |
| Natriumbenzoat | 0,40 | 0,40 | 0,40 |
| Citronensäure | 0,80 | 0,80 | 0,80 |
| vorverkleistertes hydroxypropyliertes Distärkephosphat von Wachsmaisstärke in Form lose agglomerierter Körner | 0,70 | | |
| hydroxypropyliertes Distärkephosphat von Kartoffelstärke *) | | 0,70 | |
| Parfüm | 0,80 | 0,80 | 0,80 |
| Spezifikationswerte: | | | |
| pH-Wert | 5,0 | 5,1 | 4,9 |
| Viskosität MV Stufe 1 20°C (mPas) | 14500 | 7500 | 7200 |

| | | | |
|---|---|---|---|
| *) Farinex VA 70 (Avebe/Stadex, Malmö, Schweden) | | | |

In das auf ca. 25°C temperierte Elfan NS 242 A werden nacheinander Plantaren 1200, Natriumbenzoat, Kaliumsorbat, Citronensäure, Tego Betain F 50 und die Stärkederivate - Beispiel 9 ohne - eingerührt. Wenn die Mischung homogen ist, wird das Parfüm unter Rühren zugefügt.

Gemäß Beispiel 7 resultiert ein leicht opaleszierendes viskoses Schaumbad mit angenehmen Gebrauchseigenschaften. Die Beispiele 8 und 9 ergeben im Badewasser einen grobblasigeren Schaum als Beispiel 7; Beispiel 8 ergibt zusätzlich ein negatives sandiges Hautgefühl.

Die Lagertests des Produktes gemäß Beispiel 7 bei -18°C, +4°C, Raumtemperatur (18°C bis 25°C) und + 30°C zeigten nach 1 Monat ein hinsichtlich der Spezifikationen weitgehend unverändertes Produkt.

Das Produkt gemäß Beispiel 8 führt nach 2 Tagen Lagerzeit bei Raumtemperatur zur Phasentrennung.

### Beispiele 10 bis 15:

Diese Beispiele erläutern die Herstellung einer O/W-Körperlotion. Es wurde gemäß folgender Rezeptur verfahren:

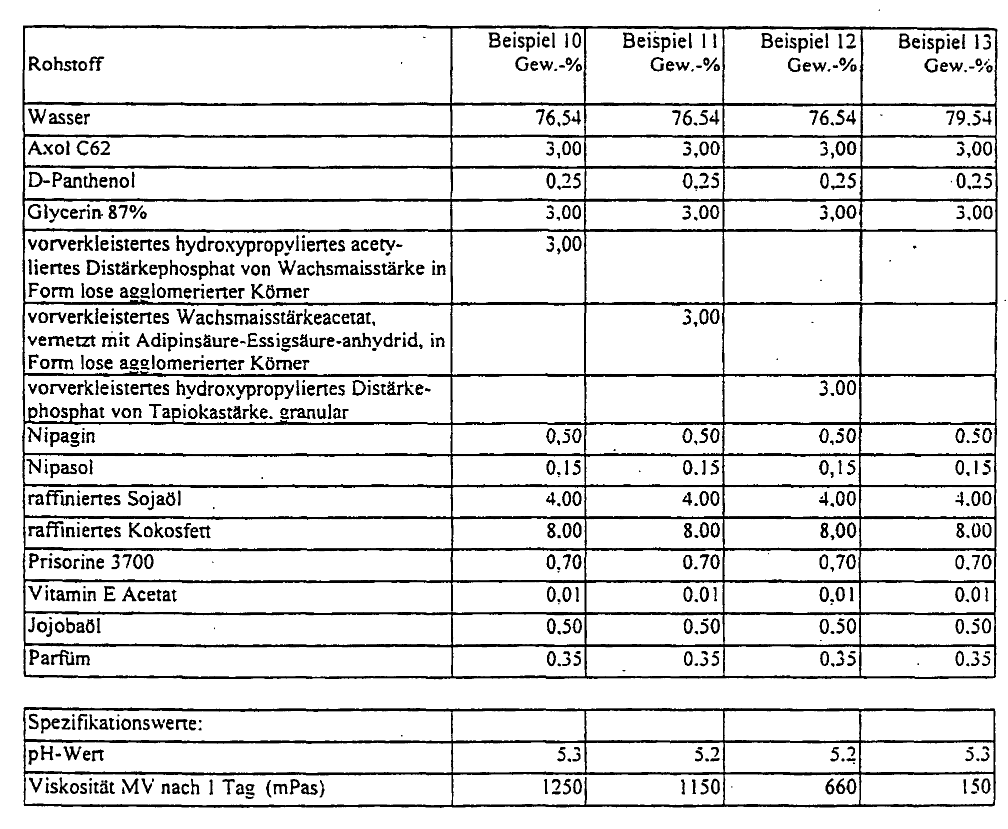

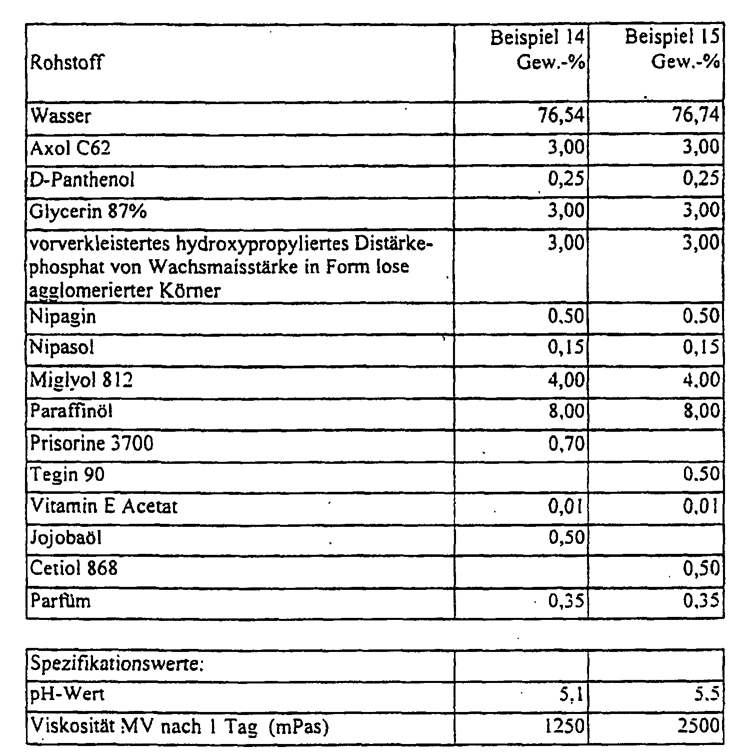

Axol C62, die Stärkederivate - Beispiel 13 ohne - Nipagin, Nipasol werden in 2/3 der Wassermenge bei 75°C unter Rühren erwärmt bis die Mischung homogen ist und dann D-Panthanol und Glycerin 87%ig dem Ansatz zugefügt. Das Kokosfett, Sojaöl, Prisorine 3700 bzw. Tegin 90 und Vitamin E-Acetat werden auf ca. 60°C erwärmt und unter Rühren der Wasserphase zugegeben. Nachdem diese Mischung homogen ist wird die restliche Menge des auf ca. 20°C temperierten Wassers unter Rühren dem Ansatz zugefügt. Bei ca. 40°C wird das Parfüm unter Rühren zugefügt. Danach wird der Ansatz mit einem Homogenisator homogenisiert und bis ca. 25°C weiter gerührt.

Bei der Anwendung auf der Haut wurde den Emulsionen gemäß der Beispiele 10, 11, 12, 14 und 15 ein hervorragendes Hautgefühl attestiert. Die Emulsion nach dem Beispiel 13 wurde als gehaltlos beurteilt.

Die Lagertests an den Beispielen 10, 11, 12, 14 und 15 bei -18°C, +4°C, Raumtemperatur (18°C bis 25 °C), + 30°C und 40°C zeigten nach 3 Monaten ein hinsichtlich der Spezifikationen weitgehend unverändertes Produkt. Die Lotion nach Beispiel 11 zeigte nach längerer Lagerung einen leichten säuerlichen Geruch.

Die Emulsion nach dem Beispiel 13 trennte sich bei allen Lagertemperaturen nach spätestens 2 Tagen.

### Beispiele 16 bis 18:

Diese Beispiele erläutern die Herstellung einer O/W-Pflegecreme. Es wurde nach folgender Rezeptur verfahren:

| Rohstoff | Beispiel 16 Gew.-% | Beispiel 17 Gew.-% | Beispiel 18 Gew.-% |
|---|---|---|---|
| Wasser | 55,735 | 55.735 | 60,735 |
| Nipagin | 0.350 | 0,350 | 0,350 |
| Nipasol | 0,150 | 0,150 | 0,150 |
| D-Panthenol | 0,200 | 0,200 | 0,200 |
| Vitamin E Acetat | 0,500 | 0,500 | 0,500 |
| Glycerin 87%ig | 5,000 | 5,000 | 5.000 |
| Sucro-Ester WE 15 | 2,000 | 2.000 | 2,000 |
| Axol C 62 | 2,000 | 2.000 | 2,000 |
| Prisorine 3700 | 0,700 | 0,700 | 0.700 |
| Parfüm | 0,100 | 0,100 | 0,100 |
| raffiniertes Kokosfett | 18,000 | 18.000 | 18.000 |
| vorverkleistertes hydroxypropyliertes Distärkephosphat von Wachsmaisstärke in Form lose agglomerierter Körner | 5,000 | | |
| hydroxypropyliertes Distärkephosphat von Tapiokastärke *) | | 5,000 | |
| raffiniertes Sojaöl | 8,000 | 8.000 | 8,000 |
| Natrium- Hyaluronat | 0,005 | 0,005 | 0,005 |
| Vitamin A-Palmitat | 0.060 | 0,060 | 0,060 |
| Ronoxan A | 0,200 | 0.200 | 0,200 |
| Jojobaöl | 2,000 | 2,000 | 2,000 |
| Spezifikationswerte: | | | |
| pH-wert | 5,1 | 5,5 | 5,3 |
| Viskosität SV Stufe 4 (20°C) | 6640 | zu niedrig viskos, nicht meßbar | zu niedrig viskos, nicht meßbar |

| | | | |
|---|---|---|---|
| *) Farinex VA 70T (Avebe/Stadex, Malmö, Schweden) | | | |

Die Stärkederivate, - Beispiel 18 ohne -, werden in Wasser bei 80°C ca. 10 min gerührt und danach Sucro-Ester WE 15 und Axol C 62 dem Ansatz unter Rühren zugeben, dann folgt D-Panthanol, Nipagin, Nipasol und Glycerin. Das Kokosfett, Sojaöl, Prisorine 3700 und Vitamin E Acetat werden separat auf ca. 60°C erwärmt und unter Rühren in die Wasserphase gegeben. Unter Rühren wird bis ca. 40°C abgekühlt und Natrium-Hyaluronat, Vitamin A-Palmitat, Ronoxan A, Jojobaöl und Parfüm unter Rühren zugegeben. Danach wird der Ansatz mit einem Homogenisator homogenisiert und bis ca. 25°C weiter gerührt.

Bei der Anwendung auf der Haut zeigt die Creme gemäß Beispiel 16 hervorragende Pflegeeigenschaften. Die Creme gemäß Beispiel 17 wurde deutlich schlechter beurteilt. Beispiel 18 war kurz nach dem Auftragen auf die Haut inhomogen.

Die Lagertests der Creme gemäß Beispiel 16 bei -18°C, +4°C, Raumtemperatur (18°C bis 25°C), + 30°C und 40°C zeigten nach 3 Monaten ein weitgehend unverändertes Produkt. Die Beispiele 17 und 18 zeigten nach 3 Tagen bei allen Testbedingungen Inhomogenitäten.

### Beispiele 19 bis 21:

Diese Beispiele erläutern die Herstellung einer alkoholhaltigen Lotion mit Deowirkung. Es wurde nach folgender Rezeptur verfahren:

| Rohstoff | Beispiel 19 Gew.-% | Beispiet 20 Gew.-% | Beispiel 21 Gew.-% |
|---|---|---|---|
| Wasser | 60.24 | 60.24 | 62.24 |
| Axol C62 | 3.00 | 3.00 | 3,00 |
| vorverkleistertes hydroxypropyliertes Distärkephosphat von Wachsmaisstärke in Form lose agglomerierter Körner | 2,00 | | |
| hydroxypropyliertes Distärkephosphat von Kartoffelstärke *) | | 2,00 | |
| D-Panthenol | 0,40 | 0,40 | 0,400 |
| Triäthylcitrat | 1,00 | 1,00 | 1,00 |
| Farnesol | 0,50 | 0,50 | 0,50 |
| Prisorine 3700 | 0,56 | 0,56 | 0,56 |
| Glycerin 87% | 3,00 | 3,00 | 3,00 |
| Parfüm | 0,80 | 0,80 | 0.80 |
| Aethanol 94,5 Gew %, | 20,00 | 20,00 | 20,000 |
| raffiniertes Sojaöl | 8,50 | 8,50 | 8.50 |
| Spezifikationswerte: | | | |
| pH-wert | 5,5 | 5,2 | 5,4 |
| Viskosität SV Stufe 4 (mPas) | 700 | 150 | 120 |

| | | | |
|---|---|---|---|
| *) Farinex VA 70 (Avebe/Stadex, Malmö, Schweden) | | | |

Die Stärkederivate - Beispiel 21 ohne- werden in Wasser bei 80°C eingestreut, ca. 10 min gerührt und danach Axol C 62 dem Ansatz unter Rühren zugeben, dann folgt D-Panthanol und Glycerin. Das Sojaöl und Prisorine 3700 werden separat auf ca. 60°C erwärmt und unter Rühren in die Wasserphase gegeben. Unter Rühren wird bis ca. 40°C abgekühlt und Aethanol, Triäthylcitrat, Farnesol und Parfüm unter Rühren zugegeben. Danach wird der Ansatz mit einem Homogenisator homogenisiert und bis ca. 25°C weiter gerührt.

Bei der Anwendung auf der Haut wurde die Lotion gemäß Beispiel 19 hinsichtlich ihrer Gebrauchseigenschaften positiv beurteilt. Die Beispiele 20 und 21 wurden als wenig gehaltvoll bzw. wäßrig bezeichnet. Beispiel 20 wurde zusätzlich als sandig beurteilt.

Die Lagertests am Beispiel 19 bei -18°C, +4°C, Raumtemperatur (18°C bis 25°C), +30°C und 40°C zeigten nach 3 Monaten ein weitgehend unverändertes Produkt.

Dagegen zeigten die Beispiele 20 und 21 nach 1 Tag Phasentrennung bei Raumtemperatur.

### Beispiele 22 bis 24:

Diese Beispiele erläutern die Herstellung einer alkoholhaltigen Creme mit Lichtschutzwirkung.

| Rohstoff | Beispiel 22 Gew.-% | Beispiel 23 Gew.-% | Beispiel 24 Gew.-% |
|---|---|---|---|
| Wasser | 47,40 | 47,40 | 49.80 |
| Axol C62 | 3,20 | 3,20 | 3,20 |
| Uvinul MS 40 | 2,00 | 2,00 | 2.00 |
| Parsol MCX | 3,00 | 3,00 | 3.00 |
| vorverkleistertes hydroxypropyliertes Distärkephosphat von Wachsmaisstärke in Form lose agglomerierter Körner | 2,40 | | |
| hydroxypropyliertes Distärkephosphat von Kartoffelstärke *) | | 2.40 | |
| D- Panthenol | 0,40 | 0,40 | 0.40 |
| Prisorine 3700 | 0,56 | 0,56 | 0,56 |
| Avocadoöl | 0,80 | 0,80 | 0.80 |
| Jojobaöl | 0,80 | 0,80 | 0.80 |
| Glycerin 87% | 3,20 | 3,20 | 3,20 |
| Parfüm | 0,24 | 0,24 | 0,24 |
| Aethanol 94,5 Gew %, | 20,00 | 20,00 | 20,00 |
| raffiniertes Kokosfett | 16,00 | 16,00 | 16.00 |
| Spezifikationswerte: | | | |
| pH-wert | 5,4 | 5,3 | 5,4 |
| Viskosität M Stufe 4 (mPas) | 3200 | 1100 | 800 |

| | | | |
|---|---|---|---|
| *) Farinex VA 70 (Avebe/Stadex, Malmö, Schweden) | | | |

Die Stärkederivate - Beispiel 24 ohne - werden in Wasser bei 80°C eingestreut, ca. 10 min gerührt und danach wird Axol C 62 dem Ansatz unter Rühren zugeben, dann folgen Uvinul MS 40, D-Panthanol und Glycerin. Das Kokosfett, Avocadoöl, Jojobaöl, Parsol MCX und Prisorine 3700 werden separat auf ca. 60°C erwärmt und unter Rühren in die Wasserphase gegeben. Unter Rühren wird bis ca. 40°C abgekühlt und Aethanol und Parfüm unter Rühren zugegeben. Danach wird der Ansatz mit einem Homogenisator homogenisiert und bis ca. 25°C weiter gerührt.

Die Gebrauchseigenschaften des Produktes gemäß Beispiel 22 wurden als gut bezeichnet.

Die Lagertests bei -18°C, +4°C, Raumtemperatur (18°C bis 25°C), + 30°C und 40°C zeigten nach 3 Monaten ein weitgehend unverändertes Produkt.

Die Gebrauchseigenschaften der Produkte gemäß Beispiel 23 und 24 wurden als wenig gehaltvoll bezeichnet. Das Beispiel 23 vermittelte zusätzlich noch einen sandigen Eindruck.

Die Produkte gemäß Beispiel 23 und 24 zeigten nach 1 Tag bereits Phasentrennung.

### Beispiele 25 bis 34:

Diese Beispiele erläutern die Herstellung einer O/W-Körperlotion. Es wurde gemäß folgender Rezeptur verfahren:

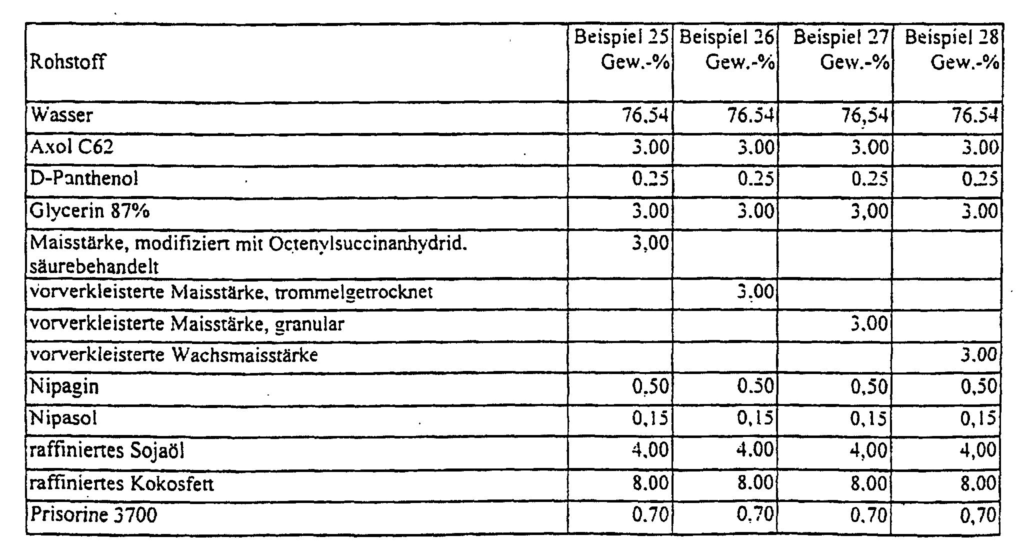

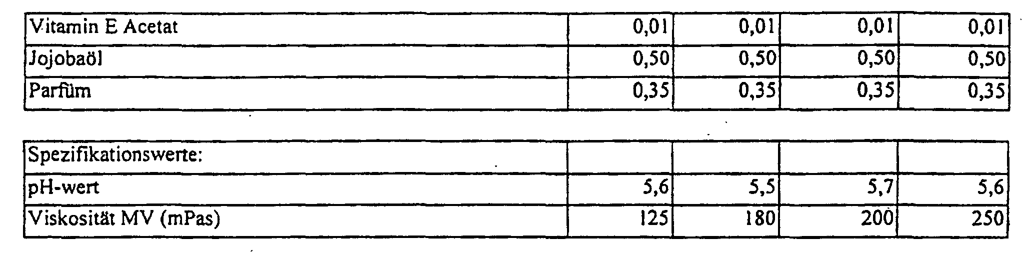

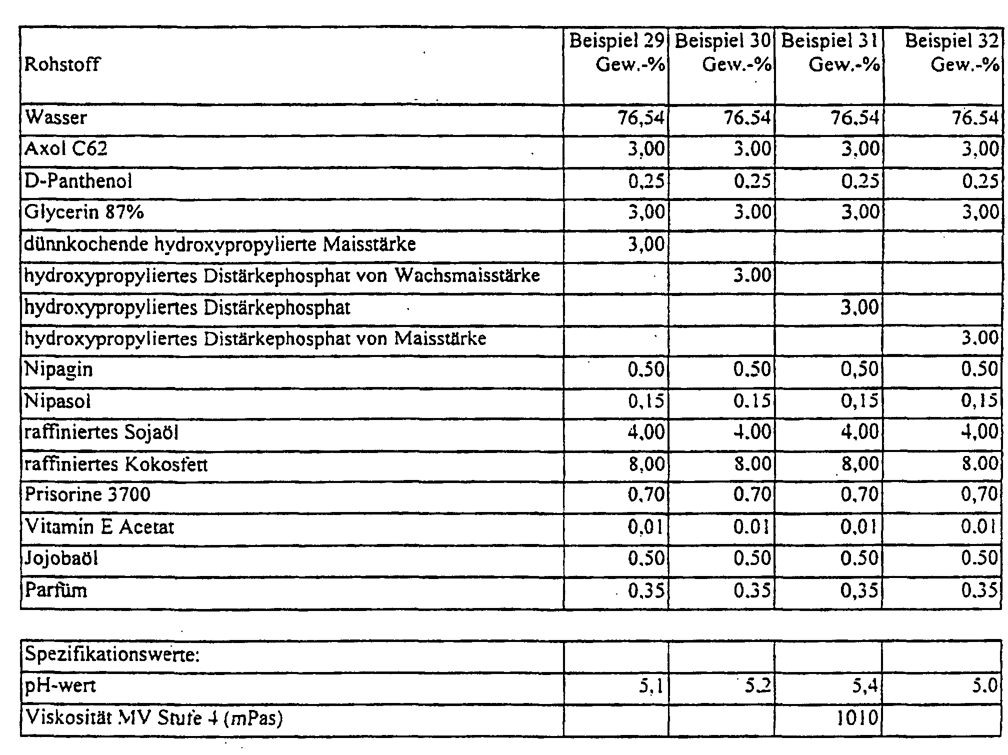

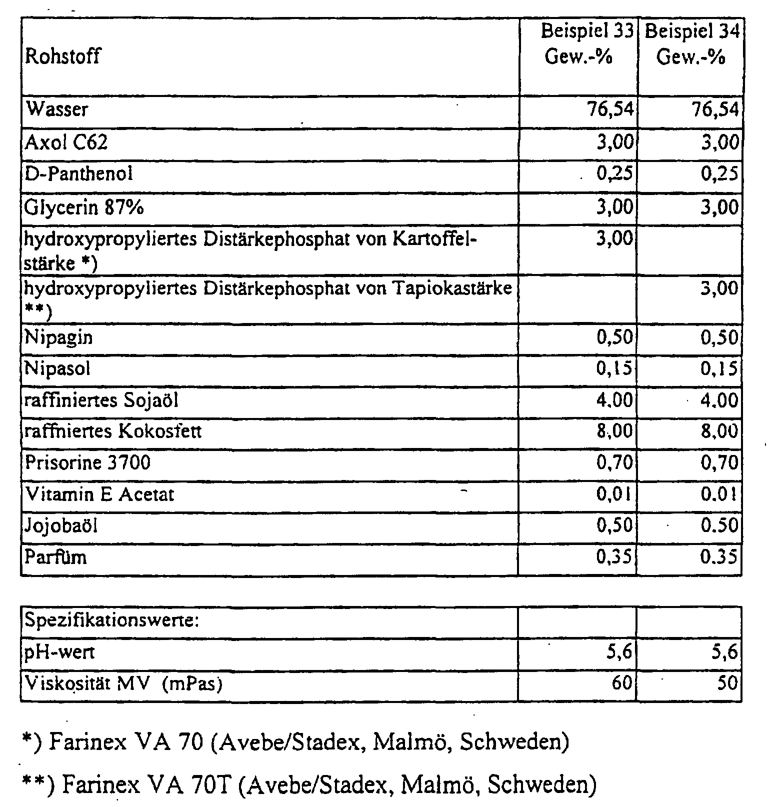

Die Herstellung erfolgte wie bei den Beispielen 10 bis 15.

Die Lagerstabilität war bei den Beispielen 25 bis 32 nicht ausreichend, es zeigte sich nach kurzer Zeit deutliche Phasentrennung. Die Ansätze aus den Beispielen 33 und 34 zeigten eine sehr geringe, für diese Produktgattung nicht brauchbare Viskosität. Nach 2 Tagen wurde eine Phasentrennung bei Raumtemperatur festgestellt. Bei Anwendungstests wurde ein sandiges Hautgefühl bemerkt.

### Beispiele 35 und 36:

Diese Beispiele erläutern die Herstellung einer O/W-Körpercreme. Es wurde gemäß folgender Rezeptur verfahren:

| Rohstoff | Beispiel 35 Gew.-% | Beispiel 36 Gew.-% |
|---|---|---|
| Wasser | 65,76 | 66,36 |
| Tego Care 215 | 3,00 | |
| Tego Care 450 | | 3,00 |
| Tegin 90 | 0,60 | |
| D-Panthenol | 0,13 | 0,13 |
| Glycerin 87% | 3,00 | 3.00 |
| vorverkleistertes hydroxypropyliertes Distärkephosphat von Wachsmaisstärke in Form lose agglomerierter Körner | 5,00 | 5,00 |
| Phenonip | 0,90 | 0,90 |
| Vitamin A-Palmitat | 0,06 | 0,06 |
| raffiniertes Sojaöl | 10,00 | 10,00 |
| raffiniertes Kokosfett | 10,00 | 10.00 |
| Prisorine 3700 | 0.70 | 0.70 |
| Vitamin E Acetat | 0,50 | 0,50 |
| Parfüm | 0,35 | 0.35 |
| Spezifikationswerte: | | |
| pH-wert | 7.0 | 7,2 |
| Viskosität MV (mPas) | 3800 | 4400 |

Vorverkleistertes hydroxypropyliertes Distärkephosphat von Wachsmaisstärke in Form lose agglomerierter Körner wird in der Wassermenge bei 80°C unter Rühren erwärmt bis die Mischung homogen ist, dann werden Phenonip, D-Panthanol und Glycerin 87%ig dem Ansatz zugefügt und die Emulgatoren Tego Care 215 bzw. Tego Care 450 eingerührt. Das Kokosfett, Sojaöl, Prisorine 3700, Tegin 90 und Vitamin E Acetat wird auf ca. 60°C erwärmt und unter Rühren der Wasserphase zugefügt. Nachdem diese Mischung homogen ist, wird der Ansatz unter Rühren abgekühlt. Bei ca. 40°C wird das Parfüm und Vitamin A-Palmitat unter Rühren zugefügt. Danach wird der Ansatz mit einem Homogenisator homogenisiert und bis ca. 25°C weiter gerührt.

Die Gebrauchseigenschaften des Produktes gemäß der Beispiele 35 und 36 wurden als gut bezeichnet.

Die Lagertests bei -18°C, +4°C, Raumtemperatur (18°C bis 25°C), + 30°C und 40°C zeigten nach 3 Monaten ein weitgehend unverändertes Produkt.

### Beispiele 37 bis 39:

Diese Beispiele erläutern die Herstellung eines Rasierschaums. Es wurde gemäß folgender Rezeptur verfahren:

| Rohstoff | Beispiel 37 Gew.-% | Beispiel 38 Gew.-% | Beispiel 39 Gew.-% |
|---|---|---|---|
| Wasser | 73,922 | 73,922 | 74,422 |
| Propan/Butan 3.5 bar | 5,000 | 5,000 | 5.000 |
| Eumulgin B2 | 1,000 | 1,000 | 1,000 |
| Glycerin 8,7% | 8,730 | 8,730 | 8,730 |
| vorverkleistertes hydroxypropyliertes Distärkephosphat von Wachsmaisstärke in Form lose agglomerierter Körner | 0,500 | | |
| hydroxypropyliertes Distärkephosphat von Tapiokastärke *) | | 0,500 | |
| Cutina FS 45 | 5.277 | 5,277 | 5.277 |
| Myristinsäure | 1.251 | 1.251 | 1,251 |
| Triäthanolamin min.99% | 3,030 | 3,030 | 3.030 |
| Luviskol K30 | 0.490 | 0,490 | 0,490 |
| Parfüm | 0.800 | 0.800 | 0.800 |
| Spezifikationswerte: | | | |
| pH-wert | 8.5 | 8.5 | 8,5 |
| Viskosität MV (mPas) | 1200 | 430 | 360 |

| | | | |
|---|---|---|---|
| *) Farinex VA 70T (Avebe/Stadex, Malmö, Schweden) | | | |

Das jeweilige Stärkederivat - Beispiel 39 ohne - wird in der Wassermenge bei 75°C unter Rühren erwärmt bis die Mischung homogen ist und dann Cutina FS 45, Myristinsäure, Eumulgin B2 und Glycerin 87 % ig dem Ansatz zugefügt. Nachdem die Fettkomponenten geschmolzen sind, wird Triäthanolamin unter Rühren dem Ansatz zugefügt. Nach Kühlen bis auf ca. 40°C wird das Parfüm und Luviskol K30 unter Rühren zugefügt. Danach wird der Ansatz mit einem Homogenisator homogenisiert und bis ca. 25°C weiter gerührt. Diese Wirkstofflösung wird in eine Aerosoldose eingefüllt, das Ventil aufgebracht und mit dem Treibgas Propan/Butan unter Druck aufgefüllt. Danach wird der Sprühkopf auf die Aerosoldose aufgebracht.

Die Gebrauchseigenschaften des Produktes gemäß Beispiel 37 wurden als gut bezeichnet. Die Produkte gemäß der Beispiele 38 und 39 wurden deutlich schlechter beurteilt.

Die Lagertests des Produktes 37 bei -18°C, +4°C, Raumtemperatur (18°C bis 25°C), +30°C und 40°C zeigten nach 3 Monaten ein weitgehend unverändertes Produkt. Die Lagertests der Produkte 38 und 39 zeigten unter den gleichen Bedingungen nach 7 Tagen Phasentrennung.

### Beispiele 40 bis 42:

Diese Beispiele erläutern die Herstellung einer W/O-Körpercreme. Es wurde gemäß folgender Rezeptur verfahren:

| Rohstoff | Beispiel 40 Gew.-% | Beispiel 41 Gew.-% | Beispiel 42 Gew.-% |
|---|---|---|---|
| Wasser | - 55,93 | 55,93 | 56,68 |
| Protegin | 20,00 | 20,00 | 20,00 |
| Bittersalz | 0,30 | 0,30 | 0,30 |
| Glycerin 87% | 2,00 | 2,00 | 2,00 |
| vorverkleistertes hydroxypropyliertes Distärkephosphat von Wachsmaisstärke in Form lose agglomerierter Körner | 0,75 | | |
| hydroxypropyliertes Distärkephosphat von Tapiokastärke *) | | 0,75 | |
| Nipagin | 0,15 | 0,15 | 0,15 |
| Nipasol | 0,05 | 0,05 | 0,05 |
| Vitamin E Acetat | 0,10 | 0,10 | 0,10 |
| raffiniertes Kokosfert | 13,00 | 13,00 | 13,00 |
| Neo PCL W/O | 5,00 | 5,00 | 5,00 |
| Butylhydroxytoluol | 0,02 | 0,02 | 0,02 |
| Hydroviton Feuchthaltefaktor | 2,00 | 0,50 | 0,50 |
| Parfüm | 0,70 | 0,70 | 0,70 |
| Spezifikationswerte: | | | |
| pH-wert | (1) | (1) | (1) |
| Viskosität SV Stufe 4 (mPas) | 21400 | 14300 | 15000 |
| (1) = nicht meßbar, da W/O System vorliegt | | | |

| | | | |
|---|---|---|---|
| *) Farinex VA 70T (Avebe/Stadex, Malmö, Schweden) | | | |

Das jeweilige Stärkederivat - Beispiel 42 ohne - Nipagin, Nipasol und Bittersalz wird in der Wassermenge bei 80°C unter Rühren erwärmt bis die Mischung homogen ist und dann Glycerin 87%ig dem Ansatz zugefügt. Das Kokosfett, Protegin, Neo PCL W/O und Butylhydroxytoluol wird auf ca. 75°C erwärmt und unter Rühren der Wasserphase zugefügt. Nachdem diese Mischung homogen ist, wird der Ansatz unter Rühren abgekühlt. Bei ca. 40°C wird das Parfüm, Hydroviton Feuchthaltefaktor und Vitamin E Acetat unter Rühren zugefügt. Danach wird der Ansatz mit einem Homogenisator homogenisiert und bis ca. 25°C weiter gerührt. Die Gebrauchseigenschaften des Produktes gemäß Beispiel 40 wurden als gut bezeichnet.

Die Lagertests bei -18°C, +4°C, Raumtemperatur (18°C bis 25°C), + 30°C und 40°C zeigten nach 3 Monaten ein weitgehend unverändertes Produkt.

Die Phasenstabilität war bei den Produkten gemäß der Beispiele 41 und 42 nicht ausreichend.

### Beispiele 43 bis 45:

Diese Beispiele erläutern die Herstellung eines Geschirrspülmittels. Es wurde gemäß folgender Rezeptur verfahren:

| Rohstoff | Beispiel 43 Gew.-% | Beispiel 44 Gew.-% | Beispiel 45 Gew.-% |
|---|---|---|---|
| Wasser | 20,90 | 20,90 | 23,90 |
| Texapon ALS | 50,00 | 50,00 | 50,00 |
| Plantaren 1200 | 5,00 | 5,00 | 5,00 |
| Rewopol SBFA | 15,00 | 15,00 | 15,00 |
| Äthanol | 5,00 | 5,00 | 5,00 |
| Citronensäure | 0,50 | 0,50 | 0,50 |
| Euxyl K400 | 0,20 | 0,20 | 0,20 |
| D-Panthenol | 0,10 | 0,10 | 0,10 |
| vorverkleistertes hydroxypropyliertes, acetyliertes Distärkephosphat von Wachsmaisstärke in Form lose agglomerierter Körner | 3,00 | | |
| hydroxypropyliertes Distärkephosphat von Tapiokastärke *) | | 3,00 | |
| Parfüm | 0,30 | 0,30 | 0.30 |
| Spezifikationswerte: | | | |
| pH - Wert | 5,5 | 5,4 | 5,7 |
| Viskosität MV (mPas) | 4200 | 1400 | 1200 |

| | | | |
|---|---|---|---|
| *) Farinex VA 70T (Avebe/Stadex, Malmö, Schweden) | | | |

In das ca. 25°C warme Wasser werden nacheinander Texapon ALS, Plantaren 1200, Rewopol SBFA, Euxyl K400, Citronensäure und die Stärkederivate - Beispiel 45 ohne - eingerührt. Nachdem die Mischung homogen ist, wird D-Panthenol, Äthanol und das Parfüm eingerührt.

Gemäß Beispiel 43 resultiert ein leicht opaleszierendes viskoses Geschirrspülmittel. Die Beispiele 44 und 45 sind deutlich niedriger viskos.

Bei der Anwendung erzeugt das Geschirrspülmittel neben einem beständigen Schaum eine gute Reinigungswirkung und ein gutes Hautgefühl. Die Beispiele 44 und 45 bilden einen deutlich grobblasigeren Schaum als das Beispiel 43.

Die Lagertests des Produktes gemäß Beispiel 43 bei -18°C, +4°C, Raumtemperatur (18°C bis 25°C) und + 30°C zeigten nach 1 Monat ein hinsichtlich der Spezifikationen weitgehend unverändertes Produkt.

Beispiel 44 zeigte nach 2 Tagen bei Raumtemperatur Phasentrennung.

### Beispiel 46:

Dieses Beispiel erläutert die Herstellung einer Zahncreme. Es wurde gemäß folgender Rezeptur verfahren:

| Rohstoff | Beispiel 46 Gew.-% |
|---|---|
| Wasser | 24,70 |
| Natrium Hydrogencarbonat | 20,00 |
| Sident 12DS | 15,00 |
| Glycerin | 15,00 |
| Sorbit/Sorbitol (70%) | 10,00 |
| Natrium Pyrophosphat | 4.00 |
| Natrium Carbonat | 2.00 |
| Texapon K1296 Pulver | 2,00 |
| vorverkleistertes hydroxypropyliertes Distärkephosphat von Wachsmaisstärke in Form lose agglomerierter Körner | 5,00 |
| Saccharin Na | 0.10 |
| Natrium Fluorid | 0,20 |
| Titandioxid | 1,00 |
| Aroma | 1.00 |
| Spezifikationswerte: | |
| pH - Wert | 9.0 |
| Viskosität MV (mPas) | 14500 |

In das ca. 25°C warme Wasser werden nacheinander Natrium Hydrogencarbonat, Natrium Carbonat, Natrium Fluorid, das Stärkederivat, Sident 12DS, Glycerin, Sorbit/Sorbitol (70%), Natrium Pyrophosphat, Texapon K1296, Saccharin Na, Titandioxid und Aroma eingerührt. Nachdem die Mischung homogen ist, wird homogenisiert.

Gemäß Beispiel 46 resultiert eine weiße, hochviskose Zahncreme.

Bei der Anwendung vermittelt die Zahncreme neben einem dichten, beständigen Schaum eine gute Reinigungswirkung und ein angenehmes Mundgefühl.

Die Lagertests des Produktes gemäß Beispiel 46 bei -18°C, +4°C, Raumtemperatur (18°C bis 25°C) und + 30°C zeigten nach 1 Monat ein hinsichtlich der Spezifikationen weitgehend unverändertes Produkt.

### Beispiel 47:

Dieses Beispiel erläutert die Herstellung einer emulgatorfreien O/W-Körperlotion. Es wurde gemäß folgender Rezeptur verfahren:

| Rohstoff | Beispiel 47 Gew.-% |
|---|---|
| Wasser | 77.700 |
| Cyclodextrin Beta W7 | 1.000 |
| Glycerin 87 % | 5,000 |
| vorverkleistertes hydroxypropyliertes Distärkephosphat von Wachsmaisstärke in Form lose agglomerierter Körner | 5,000 |
| Nipagin | 0,400 |
| Nipasol | 0,100 |
| Pionier 4656 | 5.000 |
| Vaseline | 5.000 |
| Titandioxid | 0.500 |
| Parfüm | 0.300 |
| Spezifikationswerte: | |
| pH-Wert | 6.3 |
| Viskosität MV nach 1 Tag (mPas) | 2250 |

Das Cyclodextrin, das Stärkederivat, Nipagin, Nipasol und Titandioxid werden in 2/3 der Wassermenge bei 75°C unter Rühren erwärmt, bis die Mischung homogen ist, dann wird das Glycerin zugefügt. Pionier 4656 und Vaseline werden auf ca. 60°C erwärmt und der Wasserphase zugegeben. Anschließend wird die Restmenge Wasser zugefügt. Bei 40°C wird das Parfüm zugesetzt und der Ansatz mit einem Homogenisator homogenisiert. Bei der Anwendung wurde der Körperlotion ein hervorragendes Hautgefühl attestiert. Sie war über mindestens 6 Monate stabil.

### Beispiel 48

Dieses Beispiel erläutert die Herstellung eines verdickten Haarbleichsystems. Es wurde gemäß folgender Rezeptur verfahren:

| Rohstoff | Beispiel 48 g |
|---|---|
| **Komponente A** | |
| Ammoniumpersulfat | 1,50 |
| Caliumhydrogentartrat | 1,50 |
| Natriumcarbonat | 1,50 |
| Natriumlaurylsulfat (Stepanol WA-100) | 0,50 |
| Hydroxypropyliertes Distärkephosphat von Wachsmaisstärke, vorverkleistert nach dem Verfahren der US 4 280 851 | 0,75 |
| Magnesiumhydroxid | 22,13 |
| Aluminiumhydroxid | 22,12 |
| **Komponente B** | |
| Wasserstoffperoxid, 50% | 12,00 |
| destilliertes Wasser | 88,00 |

Die Bestandteile von A wurden trocken miteinander vermischt. Die Bestandteile von B wurden miteinander vereinigt. Zum Gebrauch wurden die zwei Komponenten vereint und auf das Haar aufgetragen. Nach dem Vermischen wies das Haarbleichmittel eine Viskosität von 30.219 mPas (Brookfiedl Heliopath, 10 U/min) auf. Zum Vergleich wurde die identische Formulierung, jedoch ohne das Stärkederivat hergestellt. Das Mittel wies nach dem Vermischen eine Viskosität von 531 mPas auf.

### Beispiel 49:

Dieses Beispiel untersucht das Verhalten verschiedener Stärkeverdickungsmittel in unterschiedlichen Umgebungen. Die jeweilige Stärke wurde den folgenden wäßrigen Systemen als Dispersion mit 10% Feststoffgehalt auf eine Endkonzentration von 5 Gew.-% zugesetzt:

### Bedingung:

1) Wasser
2) 2 % NH₄OH
3) 2% NH₄OH und 1% polymeres Konditionierungsmittel (Polyquarternium 6)
4) 2% NH₄OH und 1% monomeres Konditionierungsmittel (Cetrimoniumchlorid)

Die 10%-igen Dispersionen der Kochstärken wurden durch 30 minütiges Kochen unter Rühren hergestellt. Die 10%-igen Dispersionen der vorverkleisterten Stärken wurden durch Einrühren der Stärke in Wasser bei Umgebungstemperatur hergestellt. Die Anfangsviskositäten der Stärkeformulierungen und der zeitliche Viskositätsverlauf wurden aufgezeichnet.

| Stärke | Bedingung | Viskosität,mPas, 45°C |
|---|---|---|
| Sprühgetrocknete vorverklei- | 1 | 7400 |
| sterte quarternisierte vernetzte | 2 | 2680 |
| Wachsmaisstärke | 3 | 3040 |
| | 4 | 2420 |
| | | |
| Hydroxypropyliertes Distärke- | 1 | 7892 |
| phosphat von Wachsmais- | 2 | 14092 |
| stärke, roh, hoher Substitu- | 3 | 18092 |
| tionsgrad | 4 | 7832 |
| | | |
| Trommelgetrocknetes vor- | 1 | 3100 |
| verkleistertes Hydroxypro- | 2 | 5260 |
| pyliertes Distärkephosphat von | 3 | 7940 |
| Wachsmaisstärke, mittlerer Substitutionsgrad | 4 | 1532 |
| | | |
| Sprühgetrocknetes vorver- | 1 | 3452 |
| kleistertes Hydroxypropylier- | 2 | 12840 |
| tes Distärkephosphat von | 3 | 14132 |
| Wachsmaisstärke, mittlerer | 4 | 2740 |
| Subsitutionsgrad | | |
| | | |
| mehrfachcarboxylierte Kar- | 1 | 27472 |
| toffelstärke, roh | 2 | 25500 |
| | 3 | 3732 |
| | 4 | 0 |
| | | |
| Hydroxypropylierte Wachs- | 1 | 600 |
| maisstärke, roh | 2 | 1000 |
| | 3 | 2800 |
| | 4 | 1952 |
| | | |
| keine | 1 | 0 |
| | 2 | 0 |
| | 3 | 0 |
| | 4 | 0 |

Mit Ausnahme der mehrfachcarboxylierten Stärke sind die hydroxypropylierten Distärkephosphate die wirksamsten Verdickungsmittel. Die carboxylierte Stärke wirkt nur in Abwesenheit kationischer Verbindungen stärker verdickend. Kationische Verbindungen sind mit dieser Stärke jedoch inkompatibel, da mit dem monomeren Konditionierungsmittel nach einem Tag und mit dem polymeren Konditionierungsmittel nach zwei Tagen eine Ausfällung beobachtet wird. Alle anderen Stärken zeigen mit den kationischen Verbindungen ausreichende Kompatibilität. Ferner erweist sich, daß das Vorverkleisterungsverfahren die Gebrauchstauglichkeit der Stärkederivate beeinflußt. So wirkt das trommelgetrocknete hydroxypropylierte Distärkephosphat weniger stark verdickend, als das sprühgetrocknete hydroxypropylierte Distärkephosphat. Das nicht vorverkleisterte hydroxypropylierte Dstärkephosphat wirkt wesentlich stärker verdickend als die vergleichbare nicht vorgekleisterte nicht-vernetzte Hydroxypropylstärke. Daher ist für eine optimale Wirksamkeit eine besondere Derivatisierung erforderlich. Überraschenderweise wird die verdickende Wirkung der hydroxypropylierte Distärkephosphate durch die Wirkung der Base deutlich verstärkt. Dieser Umstand macht die hydroxypropylierte Distärkephosphate zur Anwendung in Haarbehandlungsmitteln, wie Haarbleichmitteln und Haarfärbemitteln, besonders interessant. Die Viskositäten der vorverkleisterten Stärken und der Kochstärken können nicht ohne weiteres verglichen werden, weil das Ausmaß der Stärkequellung und des Stärkeabbaus durch Scherkräfte unterschiedlich sind.

In einem weiteren Versuch wurde der Einfluß des Vorverkleisterungsverfahrens, der Agglomeration, der Vernetzung und der Derivatisierung untersucht. Die verdickende Wirkung einer Reihe vorverkleisterter Stärken wurden hierzu verglichen. Die Merkmale der untersuchten Stärken und ihre verdickende Wirkung sind in der nachstehenden Tabelle angegeben.

| Ausgangsstärke | Alkylierungstyp | Vernetzung | Vorverkleisterung | Agglomeration | Bedingung | Viskosität, mPas, 45°C |
|---|---|---|---|---|---|---|
| Wachsmaisstärke | Hydroxypropyl | ja¹ | Sprühtrocknung² | ja | 1 2 | 4800 15563 |
| Wachsmaisstärke | Hydroxypropyl | ja¹ | Sprühtrocknung² | nein | 1 2 | 3738 13038 |
| Wachsmaisstärke | Hydroxypropyl | ja¹ | Trommeltrocknung | nein | 1 2 | 2738 6188 |
| Wachsmaisstärke | keine | nein | Sprühtrocknung² | ja | 1 2 | 300 500 |
| Wachsmaisstärke 10 | Acetyl | ja¹ | Sprühtrocknung² | ja | 1 2 | 3325 2563 |

| | | | | | | |
|---|---|---|---|---|---|---|
| ¹⁾ Phosphorylierung | | | | | | |
| ²⁾ Nach dem Verfahren der US 4 280 851 | | | | | | |

Die Ergebnisse zeigen, daß das Vorverkleisterungsverfahren, die Vernetzung und die Derivatisierung einen erheblichen Einfluß auf die Viskosität haben. Die Agglomeration scheint einen geringeren Einfluß auf die verdickende Wirkung zu haben, obgleich sie wegen der schnelleren Auflösung einen Anwendungsvorteil bringt. Das durch Sprühtrocknung vorverkleisterte, agglomerierte hydroxypropylierte Distärkephosphat ist das wirksamste Stärkederivat. Das sprühgetrocknete, jedoch nicht agglomerierte hydroxypropylierte Distärkephosphat ist nur geringfügig weniger wirksam. Eine größere Einbuße an Wirksamkeit ist mit der Vorverkleisterung durch Trommeltrocknung verbunden. Offensichtlich führt die mit diesem Vorverkleisterungsverfahren verbundene zusätzliche Scherung zu einem Abbau der Stärke, der ihre verdickende Wirkung verschlechtert. Die chemischen Modifizierungen sind ebenfalls für den Viskositätsaufbau kritisch. Mit der unmodifizierten sprühgetrockneten Stärke wird praktisch kein Viskositätsaufbau beobachtet. Die acetylierte vorverkleisterte Stärke ist im Vergleich zur hydroxypropylierten vorverkleisterten Stärke deutlich weniger wirksam. Insgesamt zeigen die vorstehenden experimentellen Ergebnisse, daß das wirksamste Stärkeverdickungsmittel das sprühgetrocknete hydroxypropylierte Distärkephosphat aus Wachsmaisstärke ist.

## Patentansprüche

1. Mittel zur Reinigung oder Pflege von Haut, Zähnen oder Haar oder zur Reinigung von glatten Oberflächen, das eine wäßrige Phase aufweist, die eine vorverkleisterte vernetzte Stärke, ausgewählt aus einer C2-C5-Hydroxyalkylstärke und einer C2-C18-Acylstärke, enthält.

2. Mittel nach Anspruch 1, **dadurch gekennzeichnet, daß** die Stärke (i) ein Hydroxypropyldistärkephosphat und/oder (ii) ein Distärke-C4-C18-alkan- oder -alkendioat ist.

3. Mittel nach Anspruch 1 oder 2, **dadurch gekennzeichnet, daß** die Stärke einen Amylopektingehalt von mindestens etwa 70% aufweist.

4. Mittel nach einem der Ansprüche 1 bis 3, **dadurch gekennzeichnet, daß** die Stärke durch Sprühtrocknung hergestellt worden ist.

5. Mittel nach einem der vorstehenden Ansprüche, **dadurch gekennzeichnet, daß** die Stärke weitgehend intakte Stärkekörner enthält.

6. Mittel nach Anspruch 5, **dadurch gekennzeichnet, daß** die Stärkekörner zu losen Aggregaten agglomeriert worden sind.

7. Mittel nach Anspruch 4, **dadurch gekennzeichnet, daß** die Stärke
(i) eine gleichmäßig vorverkleisterte, körnige Stärke in Form von eingedellten Kügelchen, wobei mindestens ein Hauptanteil der Stärkekörner ganz und unzerbrochen ist und die Stärkekörner in Form von lose verbundenen Agglomeraten oder einzelnen Körnern vorliegen,
(ii) eine sprühgetrocknete, nichtkörnige Stärke, die im wesentlichen nichtkristallin, nichtretrogradiert und vollständig vordispergiert ist,
oder
(iii) eine Mischung hiervon ist.

8. Mittel nach einem der vorstehenden Ansprüche, **dadurch gekennzeichnet, daß** es etwa 5 bis 98 Gew.-% wäßrige Phase aufweist.

9. Mittel nach einem der vorstehenden Ansprüche, **dadurch gekennzeichnet, daß** die wäßrige Phase etwa 0,1 bis 20 Gew.-% Stärke enthält.

10. Mittel nach einem der vorstehenden Ansprüche, **dadurch gekennzeichnet, daß** es mindestens etwa 0,3 Gew.-% grenzflächenaktive Substanzen enthält.

11. Mittel nach Anspruch 10, **dadurch gekennzeichnet, daß** die grenzflächenaktiven Substanzen anionische, amphotere, kationische und/oder nichtionische Tenside sind.

12. Mittel nach Anspruch 10 oder 11, **dadurch gekennzeichnet, daß** es sich um ein Shampoo, Duschgel, Schaumbad, eine flüssige Seife, eine Zahncreme, ein Haarnachbehandlungsmittel oder ein Handgeschirrspülmittel handelt.

13. Mittel nach einem der Ansprüche 10 bis 12, **dadurch gekennzeichnet, daß** es die grenzflächenaktive Substanz in einer Menge von etwa 7 bis 25 Gew.-% enthält.

14. Mittel nach einem der Ansprüche 10 bis 13, **dadurch gekennzeichnet, daß** der pH-Wert zwischen etwa 2,5 und 12 liegt.

15. Mittel nach Anspruch 14, **dadurch gekennzeichnet, daß** der pH-Wert zwischen etwa 4 und 9 liegt.

16. Mittel nach einem der Ansprüche 1 bis 9, **dadurch gekennzeichnet, daß** es mindestens etwa 1 Gew.-% hydrophobe Phase enthält.

17. Mittel nach Anspruch 16, **dadurch gekennzeichnet, daß** es etwa 5 bis 25 Gew.-% hydrophobe Phase enthält.

18. Mittel nach Anspruch 16 oder 17, **dadurch gekennzeichnet, daß** die hydrophobe Phase fein dispergiert ist.

19. Mittel nach einem der Ansprüche 16 bis 18, **dadurch gekennzeichnet, daß** die hydrophobe Phase flüssige oder feste Fertsäuretriglyceride, Kohlenwasserstoffe, Alkylether, Alkylethoxylate, Alkylpropoxylate, Alkylbutoxylate, Fettsäuremono- oder diester, Silicone und/oder langkettige Alkohole enthält.

20. Mittel nach einem der Ansprüche 16 bis 19, **dadurch gekennzeichnet, daß** es zusätzlichen Emulgator enthält.

21. Mittel nach Anspruch 20, **dadurch gekennzeichnet, daß** der Emulgator ein Teilester eines mehrwertigen Alkohols, ein Ethoxylat, Propoxylat und/oder Butoxylat ist.

22. Mittel nach Anspruch 20 oder 21, **dadurch gekennzeichnet, daß** der Emulgator in einer Menge von etwa 0,1 bis 10 Gew.-% vorliegt.

23. Mittel nach einem der Ansprüche 1 bis 9, **dadurch gekennzeichnet, daß** es in Form eines Gels vorliegt.

24. Mittel nach Anspruch 23, **dadurch gekennzeichnet, daß** es einen oder mehrere ein- oder mehrwertige Alkohole enthält.

25. Mittel nach Anspruch 24, **dadurch gekennzeichnet, daß** es Glycerin und/oder Ethanol enthält.

26. Mittel nach Anspruch 24 oder 25, **dadurch gekennzeichnet, daß** Alkohol in einer Menge von etwa 5 bis 25 Gew.-% vorliegt.

27. Mittel nach einem der Ansprüche 1 bis 9, **dadurch gekennzeichnet, daß** es sich um ein Haarfärbemittel oder Haarbleichmittel handelt.

28. Mittel nach Anspruch 27, **dadurch gekennzeichnet, daß** es ein Oxidationsmittel enthält.

29. Mittel nach einem der Ansprüche 27 oder 28, **dadurch gekennzeichnet, daß** es als zwei Komponenten vorliegt, wobei die eine Komponente die Stärke und die andere Komponente eine wäßrige Phase enthält.

30. Mittel nach Anspruch 29, **dadurch gekennzeichnet, daß** die die Stärke enthaltende Komponente pulverförmig ist.

31. Mittel nach einem der vorhergehenden Ansprüche, **dadurch gekennzeichnet, daß** es Zusatzstoffe in Form von Konservierungsmitteln, Duftstoffen, Aromastoffen, Lichtschutzmitteln, Antioxidantien, Vitaminen, pharmazeutischen Wirkstoffen, Füllstoffen, Sequestiermitteln, Färbemitteln, Bräunungsmitteln, zusätzlichen Verdickungsmitteln, anorganischen Salzen, pH-Regulatoren und/oder Perlglanzmitteln enthält.

## Claims

1. Composition for cleaning or caring for the skin, teeth or hair or for cleaning smooth surfaces comprising an aqueous phase, which contains a pregelatinized, crosslinked starch selected from a C₂-C₅ hydroxyalkyl starch and a C₂-C₁₈ acyl starch.

2. Composition according to claim 1, **characterized in that** the starch is (i) a hydroxypropyl di-starch phosphate and/or (ii) a di-starch-C₄-C₁₈-alkanoate or alkenoate.

3. Composition according to claim 1 or 2, **characterized in that** the starch has an amylopectin content of at least about 70%.

4. Composition according to one of the claims 1 to 3, **characterized in that** the starch is produced by spray drying.

5. Composition according to one of the preceding claims, **characterized in that** the starch contains largely intact starch granules.

6. Composition according to claim 5, **characterized in that** the starch granules are agglomerated to loose aggregates.

7. Composition according to claim 4, **characterized in that** the starch is
(i) a uniformly pregelatinized, granular starch in the form of indented spheres, in which at least a majority of the starch granules is whole and unbroken and the starch granules are in the form of loosely connected agglomerates or individual granules,
(ii) a spray dried, non-granular starch, which is substantially noncrystalline, non-retrograded and completely predispersed, or
(iii) a mixture thereof.

8. Composition according to one of the preceding claims, **characterized in that** it has about 5 to 98 wt. % aqueous phase.

9. Composition according to one of the preceding claims, **characterized in that** the aqueous phase contains about 0.1 to 20 wt. % starch.

10. Composition according to one of the preceding claims, **characterized in that** it contains at least about 0.3 wt. % surfactants.

11. Composition according to claim 10, **characterized in that** the surfactants are anionic, amphoteric, cationic and/or nonionic surfactants.

12. Composition according to claim 10 or 11, **characterized in that** it is a shampoo, shower gel, foam bath, liquid soap, dental cream, hair treatment composition or manual dishwashing composition.

13. Composition according to one of the claims 10 to 12, **characterized in that** it contains a surfactant in a quantity of about 7 to 25 wt. %.

14. Composition according to one of the claims 10 to 13, **characterized in that** the pH-value is between about 2.5 and 12.

15. Composition according to claim 14, **characterized in that** the pH-value is between about 4 and 9.

16. Composition according to one of the claims 1 to 9, **characterized in that** it contains at least about 1 wt. % hydrophobic phase.

17. Composition according to claim 16, **characterized in that** it contains about 5 to 25 wt. % hydrophobic phase.

18. Composition according to claim 16 or 17, **characterized in that** the hydrophobic phase is finely dispersed.

19. Composition according to one of the claims 16 to 18, **characterized in that** the hydrophobic phase contains liquid or solid fatty acid triglycerides, hydrocarbons, alkyl ethers, alkyl ethoxylates, alkyl propoxylates, alkyl butoxylates, fatty acid monoesters or diesters, silicones and/or long-chain alcohols.

20. Composition according to one of the claims 16 to 19, **characterized in that** it contains additional emulsifier.

21. Composition according to claims 20, **characterized in that** the emulsifier is a partial ester of a polyhydric alcohol, an ethoxylate, propoxylate and/or butoxylate.

22. Composition according to claim 20 or 21, **characterized in that** the emulsifier is present in a quantity of about 0.1 to 10 wt. %.

23. Composition according to one of the claims 1 to 9, **characterized in that** it is present in the form of a gel.

24. Composition according to claim 23, **characterized in that** it contains one or more monohydric or polyhydric alcohols.

25. Composition according to claim 24, **characterized in that** it contains glycerin and/or ethanol.

26. Composition according to claim 24 or 25, **characterized in that** alcohol is present in a quantity of about 5 to 25 wt. %.

27. Composition according to one of the claims 1 to 9, **characterized in that** the composition is a hair dying composition or a hair bleaching composition.

28. Composition according to claim 27, **characterized in that** it contains an oxidizing agent.

29. Composition according to one of claims 27 or 28, **characterized in that** it is present as two components wherein one component contains the starch and the other component contains an aqueous phase.

30. Composition according to claim 29, **characterized in that** the component containing the starch is powdry.

31. Agent according to one of the preceding claims, **characterized in that** it contains additives in the form of preservatives, perfumes, flavours, light protection agents, antioxidants, vitamins, pharmaceutical active substances, fillers, sequestrants, colouring agents, bronzing agents, additional thickeners, inorganic salts, pH-regulators and/or nacreous luster agents.

## Revendications

1. Composition pour le nettoyage ou l'entretien de la peau, des dents ou des cheveux ou pour le nettoyage de surfaces lisses, qui présente une phase aqueuse qui contient un amidon réticulé gonflant, choisi entre un (hydroxyalkyle en C₂ à C₅)-amidon et un (acyle en C₂ à C₁₈)-amidon.

2. Composition suivant la revendication 1, **caractérisée en ce que** l'amidon (i) est un phosphate d'hydroxypropyl-diamidon et/ou (ii) un (alcane ou alcène en C₄ à C₁₈)-dioate de diamidon.

3. Composition suivant la revendication 1 ou 2, **caractérisée en ce que** l'amidon a une teneur en amylopectine d'au moins 70 % environ.

4. Composition suivant l'une des revendications 1 à 3, **caractérisée en ce que** l'amidon a été produit par séchage par pulvérisation.

5. Composition suivant l'une des revendications précédentes, **caractérisée en ce que** l'amidon contient des grains d'amidon en grande partie intacts.

6. Composition suivant la revendication 5, **caractérisée en ce que** les grains d'amidon ont été agglomérés en agrégats lâches.

7. Composition suivant la revendication 4, **caractérisée en ce que** l'amidon
(i) est un amidon granuleux uniformément gonflant sous forme de sphérules bosselés, au moins la plupart des grains d'amidon étant entiers et non brisés et les grains d'amidon se présentant sous forme d'agglomérats liés de façon lâche
ou de grains individuels,
(ii) est un amidon non granuleux séché par pulvérisation, qui est principalement non cristallin, non rétrogradé et entièrement prédispersé,
ou bien
(iii) est un mélange de ces formes.

8. Composition suivant l'une des revendications précédentes, **caractérisée en ce qu'**elle présente une phase aqueuse à environ 5 à 98 % en poids.

9. Composition suivant l'une des revendications précédentes, **caractérisée en ce que** la phase aqueuse contient environ 0,1 à 20 % en poids d'amidon.

10. Composition suivant l'une des revendications précédentes, **caractérisée en ce qu'**elle contient au moins environ 0,3 % en poids de substances tensio-actives.

11. Composition suivant la revendication 10, **caractérisée en ce que** les substances tensio-actives sont des agents tensio-actifs anioniques, amphotères, cationiques et/ou non ioniques.

12. Composition suivant la revendication 10 ou 11, **caractérisée en ce qu'**elle est un shampoing, un gel de douche, un produit de bain moussant, un savon liquide, un dentifrice, un après-shampoing ou un produit pour vaisselle lavée à la main.

13. Composition suivant l'une des revendications 10 à 12, **caractérisée en ce qu'**elle contient la substance tensio-active en une quantité d'environ 7 à 25 % en poids.

14. Composition suivant l'une des revendications 10 à 13, **caractérisée en ce que** la valeur du pH se situe entre environ 2,5 et 12.

15. Composition suivant la revendication 14, **caractérisée en ce que** la valeur du pH se situe entre environ 4 et 9.

16. Composition suivant l'une des revendications 1 à 9, **caractérisée en ce qu'**elle contient au moins environ 1 % en poids de phase hydrophobe.

17. Composition suivant la revendication 16, **caractérisée en ce qu'**elle contient environ 5 à 25 % en poids de phase hydrophobe.

18. Composition suivant la revendication 16 ou 17, **caractérisée en ce que** la phase hydrophobe est finement dispersée.

19. Composition suivant l'une des revendications 16 à 18, **caractérisée en ce que** la phase hydrophobe contient des triglycérides d'acides gras, des hydrocarbures, des éthers d'alkyle, des éthoxylates d'alkyle, des propoxylates d'alkyle, des butoxylates d'alkyle, des mono-esters ou diesters d'acides gras, des silicones et/ou des alcools à longue chaîne liquides ou solides.

20. Composition suivant l'une des revendications 16 à 19, **caractérisée en ce qu'**elle contient en outre un émulsifiant.

21. Composition suivant la revendication 20, **caractérisée en ce que** l'émulsifiant est un ester partiel d'un polyalcool, un éthoxylate, un propoxylate et/ou un butoxylate.

22. Composition suivant la revendication 20 ou 21, **caractérisée en ce que** l'émulsifiant est présent en une quantité d'environ 0,1 à 10 % en poids.

23. Composition suivant l'une des revendications 1 à 9, **caractérisée en ce qu'**elle est sous forme d'un gel.

24. Composition suivant la revendication 23, **caractérisée en ce qu'**elle contient un ou plusieurs mono-alcools ou polyalcools.

25. Composition suivant la revendication 24, **caractérisée en ce qu'**elle contient du glycérol et/ou de l'éthanol.

26. Composition suivant la revendication 24 ou 25, **caractérisée en ce que** l'alcool est présent en une quantité d'environ 5 à 25 % en poids.

27. Composition suivant l'une des revendications 1 à 9, **caractérisée en ce qu'**elle est un produit capillaire de teinture ou de décoloration.

28. Composition suivant la revendication 27, **caractérisée en ce qu'**elle contient un agent oxydant.

29. Composition suivant la revendication 27 ou 28, **caractérisée en ce qu'**elle se présente sous la forme de deux composants dont l'un contient l'amidon et l'autre contient une phase aqueuse.

30. Composition suivant la revendication 29, **caractérisée en ce que** le composant contenant l'amidon est sous forme de poudre.

31. Composition suivant l'une des revendications précédentes, **caractérisée en ce qu'**elle contient des additifs sous forme de conservateurs, de parfums, d'arômes, d'agents de protection solaire, d'anti-oxydants, de vitamines, de substances pharmaceutiques actives, de charges, de séquestrants, de colorants, de produits bronzants, d'épaississants additionnels, de sels inorganiques, de régulateurs de pH et/ou de substances produisant un effet nacré.
